(19) Europäisches Patentamt
European Patent Office
Office européen des brevets

(11) Publication number: **0 132 881**
**A1**

# (12) EUROPEAN PATENT APPLICATION

(21) Application number: 84201035.7

(22) Date of filing: 11.07.84

(51) Int. Cl.[4]: C 07 C 131/00
C 07 C 149/14, C 07 C
C 07 C 147/14, C 07 D 303/22
C 07 D 317/28, A 01 N 37/52

(30) Priority: 15.07.83 JP 127825/83
07.10.83 JP 187004/83
07.10.83 JP 187005/83
21.02.84 JP 29504/84
21.02.84 JP 29505/84
21.02.84 JP 29506/84
25.02.84 JP 35020/84
09.04.84 JP 69129/84

(43) Date of publication of application:
13.02.85 Bulletin 85/7

(84) Designated Contracting States:
AT BE CH DE FR GB IT LI NL

(71) Applicant: NIPPON SODA CO., LTD.
2-1, Ohte-machi 2-chome
Chiyoda-ku, Tokyo, 100(JP)

(72) Inventor: Hayakawa, Koichi
1133-1, Kokufuhongo Ohiso-machi
Naka-gun Kanagawa-ken(JP)

(72) Inventor: Nishikawa, Hiroaki
2191, Ohiso-machi Naka-gun
Kanagawa-ken(JP)

(72) Inventor: Hashimoto, Sho
5579-1, Tsutsumi
Chigasaki-shi Kanagawa-ken(JP)

(74) Representative: van der Beek, George Frans et al,
Nederlandsch Octrooibureau Johan de Wittlaan 15 P.O.
Box 29720
NL-2502 LS 's-Gravenhage(NL)

## (54) Formamidoxime derivatives.

(57) A compound having the formula

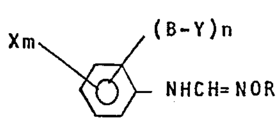

wherein X represents same or different substituent(s) selected from a group consisting of halogen, nitro, cyano, formyl, $C_{2\sim8}$ alkylcarbonyl, carboxy, $C_{2\sim8}$ alkoxycarbonyl, $C_{3\sim8}$ alkenyloxycarbonyl, $C_{3\sim8}$ alkynyloxycarboxyl, carbamoyl, $C_{2\sim6}$ alkylcarbamoyl, hetrocyclic radical containing oxygen, and a satulated or unsatulated $C_{1\sim6}$ hydrocarbon radical which may be substituted by cyano, hydroxy, halogen, $C_{1\sim6}$ alkoxy, $C_{1\sim6}$ alkoxy substituted by $C_{1\sim6}$ alkoxy, $C_{2\sim6}$ alkenyloxy, $C_{2\sim6}$ alkynyloxy, $C_{3\sim6}$ alkynyloxycarbonyloxy, $C_{1\sim6}$ alkylthio, $C_{1\sim6}$ alkylsulfinyl, $C_{1\sim6}$ alkylsulfonyl, amino substituted by $C_{1\sim6}$ alkyl, hydroxyimino, $C_{1\sim6}$ alkoxyimino and/or $C_{2\sim8}$ or alkoxycarbonyl; and

-B- represents -O-, -S-, -SO-, $-SO_2-$ or -N(R')- (R' is hydrogen or $C_{1\sim6}$ alkyl); and

Y represents hydrogen or same or different substituent(s) consisting of satulated or unsaturated $C_{1\sim6}$ hydrocarbon radicals which may be substituted by halogen, cyano, $C_{3\sim6}$ cycloalkyl, $C_{2\sim8}$ alkylcarbonyloxy, $C_{2\sim8}$ alkylcarbonyl, $C_{2\sim8}$ alkoxycarbonyl, hydroxy, $C_{1\sim6}$ alkoxy, $C_{1\sim6}$ alkylthio, ureido or heterocyclic radical containing oxygen; and a part of $-(B-Y)_n$ represents bi-substitutive modified radical $-(B-Y)-$ selected from a group consisting of $C_{1\sim3}$ alkylenedioxy which may be substituted by $C_{1\sim6}$ alkoxy, $-O-(CH_2)_l-O(CH_2)_{l'}-$ and $-O-(CH_2)_l-O-CO-$(each of l and l' is a integer from 1 to 3); and each of m and n represents an integer from 0 to 5 with the proviso that $0 \leq m+n \leq 5$ and the substituent shown above takes "one", except the bisubstitutive modified -B-Y type radical $-(B-Y)-$ which takes "two"; and

R represents a substituent selected from a group consisting of satulated or unsaturated $C_{1\sim18}$ hydrocarbon radicals which may be substituted by halogen, cyano, $C_{1\sim6}$ alkoxy, $C_{1\sim6}$ alkylthio, $C_{1\sim6}$ alkoxycarbonyl; and with the proviso that "C number ~ number" represents the range of total carbon number of the substituent or radical directly following thereto;

or a salt thereof with an organic or inorganic acid; or a complex thereof with a metal salt; and a fungicide, and insecticide and/or an acaricide containing said compound, salt, and/or complex.

EP 0 132 881 A1

# S P E C I F I C A T I O N

## Formamidoxime Derivatives

The present invention relates to a novel compound of formamidoxime derivatives and a salt or complex thereof, and fungicidal, insecticidal and/or acaricidal compositions in a form of mixture, emulsion or solution of such compound, salt or complex with or in inert carrier(s) or solvent(s), and a process for the preparation of such compound, salt or complex.

According to a first aspect of the present invention, there is provided a compound having the formula

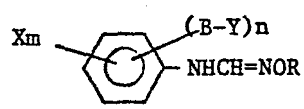 [I]

wherein X represents same or different substituent(s) selected from a group consisting of halogen, nitro, cyano, formyl, $C_{2\sim8}$ alkylcarbonyl, carboxy, $C_{2\sim8}$ alkoxycarbonyl, $C_{3\sim8}$ alkenyloxycarbonyl, $C_{3\sim8}$ alkynyloxy-carboxyl, carbamoyl, $C_{2\sim6}$ alkylcarbamoyl, hetrocyclic radical containing oxygen, and satulated or unsatulated $C_{1\sim6}$ hydrocarbon radicals which may be substituted by cyano, hydroxy, halogen, $C_{1\sim6}$ alkoxy, $C_{1\sim6}$ alkoxy substituted by $C_{1\sim6}$ alkoxy, $C_{2\sim6}$ alkenyloxy, $C_{2\sim6}$ alkynyloxy, $C_{3\sim8}$ alkynyloxycarbonyloxy, $C_{1\sim6}$ alkylthio, $C_{1\sim6}$ alkylsulfinyl, $C_{1\sim6}$ alkylsulfonyl, amino substituted by $C_{1\sim6}$ alkyl, hydroxyimino, $C_{1\sim6}$ alkoxyimino and/or $C_{2\sim8}$ alkoxycarbonyl; and

-B- represents -O-, -S-, -SO-, $-SO_2-$ or $-\overset{R'}{\overset{|}{N}}-$ (R' is hydrogen or $C_{1\sim6}$ alkyl); and

Y represents hydrogen or same or different substituent(s) selected from a group consisting of satulated or unsaturated $C_{1}\sim_{6}$ hydrocarbon radicals which may be substituted by halogen, cyano, $C_{3}\sim_{6}$ cycloalkyl, $C_{2}\sim_{8}$ alkylcarbonyloxy, $C_{2}\sim_{8}$ alkylcarbonyl, $C_{2}\sim_{8}$ alkoxycarbonyl, hydroxy, $C_{1}\sim_{6}$ alkoxy, $C_{1}\sim_{6}$ alkylthio, ureido and/or heterocyclic radical containing oxygen; and

a part of $\leftarrow$B-Y)n represents bi-substitutive modified radical $\leftarrow$B-Y$\rightarrow$ selected from a group consisting of $C_{1}\sim_{3}$ alkylenedioxy which may be substituted by $C_{1}\sim_{6}$ alkoxy, $-O-(CH_2)_{\ell}-O(CH_2)_{\ell'}-$ and $-O-(CH_2)_{\ell}-O-CO-$ (each of ℓ and ℓ' is a integer from 1 to 3); and

each of m and n represents an integer from 0 to 5 with the proviso that $0 \leq m+n \leq 5$ and the substituent shown above takes "one", except the bi-substitutive modified -B-Y type radical $\leftarrow$B-Y$\rightarrow$ which takes "two"; and

R represents a substituent selected from a group consisting of satulated or unsatulated $C_{1}\sim_{18}$ hydrocarbon radicals which may be substituted by halogn, cyano, $C_{1}\sim_{6}$ alkoxy, $C_{1}\sim_{6}$ alkylthio, $C_{1}\sim_{6}$ alkoxycarbonyl; and

with the proviso that "C number $\sim$ number" represents the range of total carbon number of the substituent or radical directly following thereto;

or a salt thereof with an organic or inorganic acid; or a complex thereof with a metal salt.

According to a second aspect of the present invention, there is provided a fungicidal, insecticidal and/or acaricidal composition comprising an effective amount of the compound having the formula [I] and inert additive(s) and/or carrier(s).

In the event of cultivating plants for agriculture or horticulture, numerous preventive chemicals are used to control the injurious fungi, insects and/or mites to the plants or crops, however, in some occasion, the preventive effects thereof are not sufficient or plants or crops are polluted by phytotoxicity thereof or they have strong poisonous influence to human, animal or fish, so that a big number of preventive chemicals can not be said satisfactory chemicals or become inadequate to commercially apply on the plants.

Consequently, appearance of preventive chemicals which are free from above drawbacks and can be used in safety is eagerly required.

The inventors, paying attentions to above drawbacks, carried out investigation on numerous compounds, and as the results, they discovered that the compound having the formula [I] indicates a superior fungicidal effect to the various plant disease fungi.

Formamidoxim derivatives having hydrogen atom at the place of R of the formula [I] are disclosed in U.S.P. 4,237,168, however, in the specification of which any fungicidal activity of such compound is not shown and the insecticidal activity is solely appeared.

We have very famous and world widely by prevailing fungicides since about 1970 developed for agriculture and horticulture so-called "benzimidazole-thiophanate series fungicidal compounds" which include benomyl [methyl 1-(butylcarbamoyl)-benzimidazole-2-yl-carbamate], fuberidazole [2-(2-furyl) benzimidazole], thiabendazole [2-(4-thiazolyl)benzimidazole], carbendazim [methyl benzimidazole-2-yl-carbamate], thiophanate methyl [1,2-bis(3-methoxycarbonyl-2-thioureido)benzene] and thiophanate [1,2-bis(3-ethoxycarbonyl-2-thioureido)benzene] or the like, which indicate a suprerior exterminating effect to the various disease germs as the

parasites of the agriculture and horticulture plants and crops and served grately to multiply production of the crops (hereinafter called as "benzimidazole-thiophanate series compounds").

But if the same fungicides as above have been sprayed continuously on the plants and crops at the same field for a long period, the fungi such treated will obtain a resistance against such benzimidazole-thiophanate series compounds (such resistant fungi are hereinafter called as "Chemical Resistant Fungi") and will prevail increasingly and thereby the preventive effect of the fungicides will be deteriorated to a status that the said fungicides can not be practically used.

In such event, the farmer, in other words the user of said fungicides, will be obliged to use another effective fungicide to exterminate the chemical resistant fungi, if any.

However, it is regretifully that any superior fungicide comparatively same efficacious as benzimidazole-thiophanate series fungicides before Chemical Resistant Fungi had become prevail has not yet appeared in the market so that it is difficult to secure adequate preventive means against plant diseases.

The inventors, taking into consideration of above circumstances, eagerly synthesized a great number of novel compounds and thoroughly evaluated their fungicidal activity against Chemical Resistant Fungi, because if they indicate strong selective fungicidal activities against Chemical Resistant Fungi, the high preventive effect can be expected for the plants in the field where Chemical Resistant Fungi are prevailing.

As the results, they discovered that a compound having the formula [I] and having at least one -B-Y radical (such compound is hereinafter called as "B-Y

compound") has a selective preventive effect to the plant disease caused by Chemical Resistant Fungi. In other words, they found that the compound set forth above has the selective fungicidal activity against Chemical Resistant Fungi.

Contrary to a superior high fungicidal activity against Chemical Resistant Fungi, B-Y compound does not or hardly show the preventive effect against injurious diseases caused by fungi sensitive to benzimidazole-thiophanate series compounds (such fungi are hereinafter called as "Chemical Sensitive Fungi") as manifested in the later Tests. Therefore, it should be stated that B-Y compound has a highly sensitive selectivity on the fungicidal activity to Chemical Resistant Fungi.

As for the intensity of the selective fungicidal activity to Chemical Registant Fungi of B-Y compound, the compound having -B-Y radical at 3 and/or 4-positions of phenyl group thereof has a very strong said selective fungicidal activity, however, the compound having -B-Y radical solely at the 2-position seems to have rather weak activity. In case that -B- is -O-, said selective activity would come the strongest in comparison with the corresponding B-Y compound having a fragment -B- other than -O-.

Taking into account of the conditions of the practically applying fields in which Chemical Resistant Fungi and Chemical Sensitive Fungi are miscibly spreading on plants, mixtures having a numerous kind of combination of a compound selected from the B-Y compounds and a compound selected from benzimidazole-thiophanate series compounds (hereinafter called as "Mixed Chemicals") are tested on the preventive effect against various diseases of plants in comparison with single compound before such conbination, and it was discovered and confirmed the unexpectedly superior efficacy of such combination for the prevention of plant

deseases caused by infection of either Chemical Resistant Fungi or Chemical Sensitive Fungi or the mixture thereof (hereinafter called as "Mixed Fungi").

Therefore, now it can be stated that the plant deseases almost uncurable because of infection of Mixed Fungi have become able to be completely curable by applying a mixed fungicide of such combination of two specific kinds of active ingredients as shown above, i. e. Mixed Chemicals.

Furthermore, it was extremely surprising that Mixed Chemicals showed unexpectedly superior efficacy for exterminating Chemical Resistant Fungi as well as Chemical Sensitive Fungi, those of which are separately cultivated. For example, Mixed Chemicals shows 5 to 10 times of fungicidal activity against either kind of fungi as shown above on the basis of activity of B-Y compounds against Chemical Resistant Fungi and activity of Benzimidazole-Thiophanate Series Compounds against Chemical Sensitive Fungi.

Accordingly, Mixed Chemicals of this invention should express their superior fungicidal power at miscellaneous conditions of fungi in the practical agricultural or horticultural fields.

For example, Cercospora leaf spot (Cercospora beticola) of beet, Cercospora leaf spot (Cercospora arachidicola) and leaf spot (Cercospora personata) of peanut, powdery mildew (Sphaerotheca fuliginea), Gummy stem blight (Mycosphaerella melonis), Sclerotinia rot (Sclerotinia sclerotiorum), gray mould (Botrytis cinerea) and scab (Cladosporium cucumerinum) of cucumber, gray mould (Botrytis cinerea) and leaf mould (Cladosporium fulvum) of tomato, gray mould (Botrytis cinerea), black rot (Corynespora melongenae) and powdery mildew (Erysiphe

cichoracearum) of eggplant, gray mould (Botrytis cinerea) and powdery mildew (Sphaerotheca humuli) of strawberry, gray-mould neck rot (Botrytis alli) and gray mould (Botrytis cinerea) of onion, powdery mildew (Erysiphe cichoracearum) of tobacco, Sclerotinia rot (Sclerotinia sclerotiorum) and gray mould (Botrytis cinerea) of kidney bean, powdery mildew (Podosphaera leucotricha), scab (Venturia inaequalis) and blossom blight (Sclerotinia mali) of apple, powdery mildew (Phyllactinia kakicola), anthracnose (Gloeosporium kaki) and Angular-leaf spot (Cercospora kaki) of persimmon, brown rot (Sclerotinia cinerea) of peach and cherry fruit, gray mould (Botrytis cinera), powdery mildew (Uncinula necator) or ripe rot (Glomerella cingulata) of grape, scab (Venturia nashicola) of pear, gray blight (Pestalotia theae) and anthracnose (Colletotrichum theae-sinensis) of tea-plant, scab (Elsinoe fawcetti), blue mould (Penicillium italicum) and common green mould (Penicillium digitatum) of orange, powdery mildew (Erysiphe graminis f. sp. hordei), eye spot (Pseudocercosporella herpotrichoides) and Fusarium snow blight (Fusarium nivale) of barley, powdery mildew (Erysiphe graminis f. sp. tritici) of wheat or the like may be exterminated.

Further, as metioned above, the farmer, in other words the user of preventive fungicides, was encountered to the difficulties incleased by prevailing Chemical Resistant Fungi and become unable to secure the prevention of disease injury of plants and crops caused by Chemical Resistant Fungi or a mixture of Chemical Resistant Fungi and Chemical Sensitive Fungi, i.e. Mixed Fugni.

Since about 1980, fungicides containing cyclic imide series compounds such as N-(3',5'-dichlorophenyl)-1,2-dimethylcyclopropane-1,2-dicarboxyimide, 3-(3',5'-dichlorophenyl)-2-isopropylcarbamoylimidazolidine-2,4-dione or 3-(3',5'-dichlorophenyl)-5-methyl-5-vinyloxazolidine-2,4-dione have been sold in the market.

The cyclic imide series compounds indicate a strong fungicidal activity to Chemical Resistant Fungi as well as Chemical Sensitive Fungi. Especially, they indicate a surperior fungicidal activity to gray mould (Botrytis cinerea) of grape or vegetables and brown rot (Sclerotinia cinerea) of peach or cherry fruits.

In so far as those disease injuries, the farmers obtained tentative success on taking counter measure for prevention of Chemical Resistant Fungi by utilizing the fungicides containing cyclic imide series compounds. However, fungi resistant to cyclic imide series compounds also appeared and the preventive effect of such fungicides to the fungous injuries of plants consequently had become deteriorate.

Under the consideration to the above cirumstances, the inventors adopted four kinds of combined fungi (Botrytis cinerea), i. e. fungi sensitive to benzimidazole-thiophanate series compounds and sensitive to cyclic imide series compounds (hereinafter designated as "BT/S & CI/S"), fungi sensitive to benzimidazole-thiophanate series compounds and resistant to cyclic imide series compounds (hereinafter designated as "BT/S & CI/R"), fungi resistant to benzimidazole-thiophanate series compounds and sensitive to cyclic imide series compounds (hereinafter designated as "BT/R & CI/S") and fungi resistant to benzimidazole-thiophanate series compounds and resistant to cyclic imide series compounds (hereinafter designated as "BT/R & CI/R") (hereinafter collectively called as "Combined Fungi") for the test to confirm the fungicidal activity of the compounds of this invention against Combined Fungi.

As the results, it was found that the compound of this invention having an extremely highly selective fungicidal activity to fungi having property of BT/R

always has extremely highly fungicidal activity to the Combined Fungi having BT/R property regardless whether the fungi are resistant or sensitive to cyclic imide series compounds or so (other fungicidal compounds).

Contrary to the above mentioned facts, notwithstanding, B-Y compound indicates also preventive effect to the plant diseases such as rice blast, cucumber downy mildew or the like, irrespective of whether the fungi causing above diseases are resistant or sensitive to benzimidazole-thiophanate series compounds.

A compound having the formula [I] further has an insecticidal and/or acaricidal activity to injurious insects such as Army worm, Aphids species or Green rice leafhopper etc. and the injurious acari such as two spotted spider mite etc.

According to a third aspect of the invention, there are provided processes for the preparation of the compound having the formula [I], comprising the step of reacting as illustrated by the following equations (1), (3) and (4).

X, -B-, Y, R, m and n used hereinafter have the same meanings as shown in the explanation of the formula [I] first appearing herein.

1.

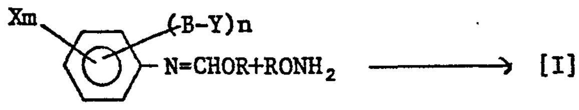
..... (1)

[II] [III]

wherein r is $C_1 \sim_4$ alkyl.

(hereinafter called as "Manufacturing Process A")

Formimidates having the formula [II] is reacted with hydroxyamines having the formula [III] in an inert organic solvent. As the inert organic solvent, a polar solvent such as lower alcohols, ethers, esters, haloganated hydrocarbons etc. may be used. The reaction is generally carried out at a temperature in a range from room temperature to a boiling point of the used solvent for 0.1 ∿ 1.0 hour. The formimidates having the formula [II] may be prepared by the following reaction equation (2) in which an aniline corresponding to the formimidate is reacted with an ortho $C_1 \sim_4$ alkyl formate.

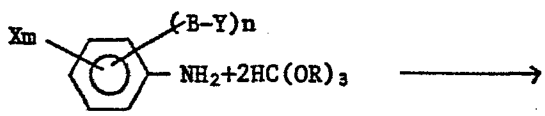
[II] ..... (2)

[IV]

(hereinafter called as "Raw Material Process")

2. (a)

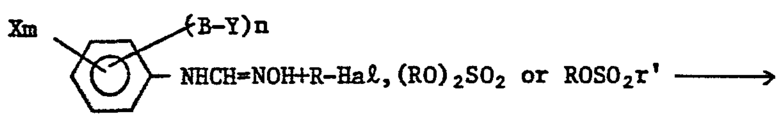
[I] ..... (3)

[V] [VI] [VII] [VIII]

wherein Hal is halogen and r' is $C_1 \sim_4$ alkyl or phenyl which may be substituted by methyl.

(hereinafter called as Manufacturing Process B-a)

(b)

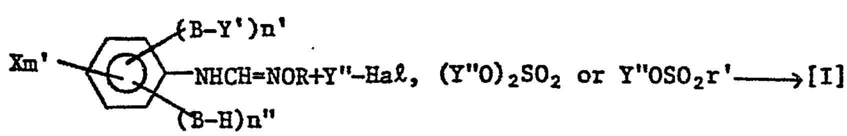
[I]' ..... (4)

[X] [XI] [XII]

[IV]

(hereinafter called as Manufacturing Process B-b)

wherein Y" represents same or different substituent(s) consisting of satulated or unsaturated $C_1\sim_6$ hydrocabon radical(s) which may be substituted by halogen, cyano, cycloalkyl, alkylcarbonyloxy alkylcarbonyl, alkoxycarbonyl, hydroxy, alkoxy, alkylthio, ureido and/or heterocyclic radical containing oxygen; and

m' represents an integer from 0 to 4;

n' represents an integer from 1 to 5;

n" represents an integer from 0 to 4, with the proviso that $1 \leq m'+n'+n'' \leq 5$.

The raw material having the formula [V] or [IX] may be prepared in accordance with (mutatis mutandis) by Manufacturing Process A, in which R is hydrogen (in case of compound [V]) or Y is hydrogen (in case of compound [IX]).

The compound having the formula [V] or [IX] is reacted with halide compound, sulfate compound or sulfonate compound having the formula [VI], [VII], [VIII], [X], [XI] or [XII].

The reaction is generally carried out in an inert organic solvent in the presence of an acid binder at a temperature in a range from 0°C to a boiling point of the used solvent for 0.5 to 3 hours.

As the inert organic solvent, a polar organic solvent such as acetone, dimethylformamide, tetrahydrofuran, acetonitrile, chloroform, toluene etc. may be used.

As the acid binder, an organic or inorganic base, for example, sodium hydride, triethylamine, pyridine, sodium hydroxide, potassium hydroxide, sodium carbonate potassium carbonate or the like may be used.

Further, in Raw Material Process and the following Manufacturing Process A, if the starting material compound (IV) has the substituent of OH at 2 or 6 position of the phenyl radical, the reactions in those processes may be partially carried out according to the following equations:

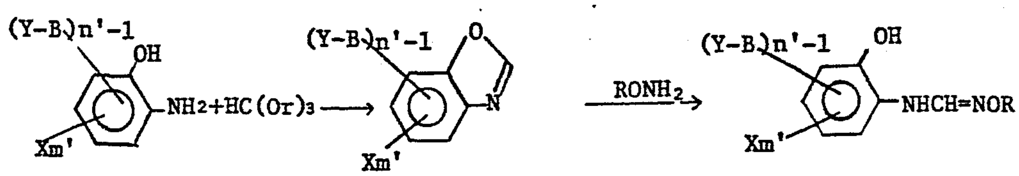


For the producing the organic or inorganic acid salt of the compound or metal salt complex of the compound having formula [I], the compound is reacted with an organic or inorganic acid or metal salt in an inert organic solvent such as chloroform, ether, acetone, acetonitrile, benzene etc. at room temperature to obtain the salt or complex as preciptation.

As the inorganic acid, hydrochloric acid, hydrobromic acid, phosphoric acid, nitric acid or the like, and as the organic acid, acetic acid, oxalic acid, maleic acid, succinic acid, fumaric acid, tartaric acid, citric acid, salicylic acid, sorbic acid, lactic acid, p-toluene sulfonic acid, methane sulfonic acid 1,5-naphthalene-disulfonic acid, picric acid, phenylphosphonic acid or the like may be utilized.

In the case of the metal salt complex, as the cation, aluminium, silicon, manganese, iron, cobalt, nickel, copper, zinc, cadmium, tin, mercury, lead or the like, and as the counter anion, the anion of chlorine, bromine, iodine,

sulfonic acid, phosphoric acid, nitric acid, carbonic acid, oxalic acid, citric acid or the like may be utilized.

The chemical structure of the compound is determined by the results of spectrum analyses of IR., NMR and/or Mass, and other analyses, if necessary.

The mol ratio of acid or metal salt to the compound in the salt or complex of this invention is determined by the elementary analysis or another method in which the salt or complex is dissolved in water and the dissociated free compound having formula [I] is filtered off and the remaining filrate is treated with a chelate titration, a neutralizing titration or an oxidative-reductive titration to determine said mol ratio of acid or metal salt.

The compound having the formula [I] has the following tautomeric forms as shown by the formulae [I] and [I-t] and each compound corresponding to each tautomeric structure should be consisting of "E isomer" and "Z isomer", thus the present invention contains all those isomers and the mixture thereof.

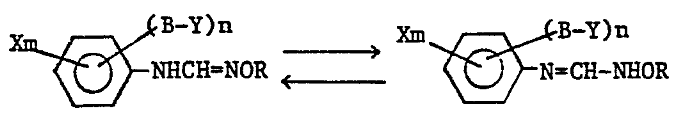

[I] [I-t]

The following Examples illustrate this invention. However, the scope of the invention shall not be limitted to those.

<u>Example 1</u> (Manufacturing Process A)

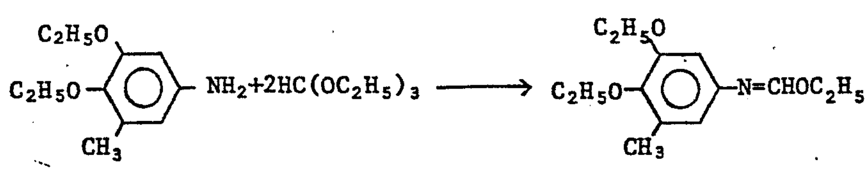

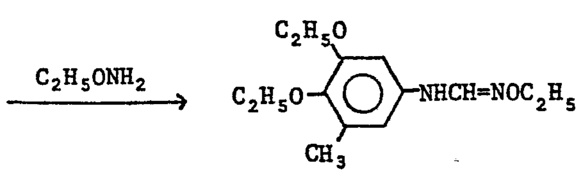


(Compound No. 250) :

2.6 g of 3,4-diethoxy-5-methylaniline, 3.9 g of ortho ethyl formate and 30 ml of ethyl acetate were mixed under stirring, and the resulting solution was heated to 70 ∿ 80°C. Ethanol produced by the reaction process was distilled off as the azeotropic mixture with ethyl acetate at atmospheric pressure.

When the distillation came to about the end point, the reaction temperature was raised to 100°C, and further, the distillation at the same temperature under such heating and stirring was continued for about 1 hour.

Then, an excess amount of ortho ethyl formate was distilled off under a reduced pressure of 15 ∿ 20 mm/Hg and a bath temperature of 50 ∿ 60°C, and 3.2 g of crude intermediate material of ethyl N-(3,4-diethoxy-5-methylphenyl)-formimidate was obtained as residue.

Said crude intermediate material was dissolved in 20 ml of chloroform, and 1.95 g of aqueous solution containing 45% of ethoxyamine was added to the solution at room temperature under stirring. After stirring for 1 hour, resulting solution was washed with water and dried with anhydrous magnesium sulfate, and then the chloroform was distilled off from the filtrate of the dried solution.

The crystals thus obtained were purified by recrystallization from a mixed solvent of n-hexane and ethyl acetate (9:1 v/v) to yield 2.8 g of objective material of N-(3,4-diethoxy-5-methylphenyl)-N'-ethoxy-formamidine (m.p. 89 ∿ 91°C).

<u>Example 2</u> (Manufacturing Process B-b)

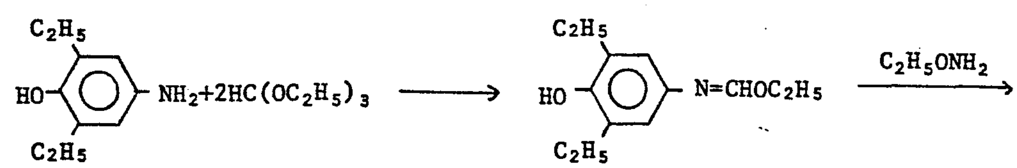

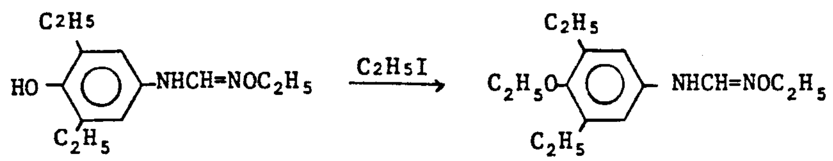


(Compound No. 135) :

27.9 g of 4-aminc-2,6-diethylphenol, 50.0 g of ortho ethyl formate and 100 ml of ethyl acetate were mixed under stirring and the resulting solution was heated to 70 ∿ 80°C. Ethanol produced by the reaction process, was distilled off as the azeotropic mixture with ethyl acetate at atmospheric pressure.

When the distillation came to about the end point, the reaction temperature was raised to 100°C and further, the distillation at the same temperature under such heating and stirring was continued for 1 hour.

Then, an excess amount of ortho ethyl formate was distilled off under a reduced pressure of 15 ∿ 20 mmHg and a bath temperature of 50 ∿ 60°C and 37.3 g of crude intermediate material of ethyl N-(3,5-diethyl-4-hydroxyphenyl)-formimidate was obtained as residue.

Said crude intermediate material was dissolved in 150 ml of methanol and 11.2 g of ethoxyamine was dropped into the solution at room temperature under stirring. After stirring for about 3 hours, the methanol disstilled off and the crystals thus obtained were washed with a mixed solvent of ether and n-hexane (1:1 v/v) to yield 37.8 g of N-(3,5-diethyl-4-hydroxyphenyl)-N'-ethoxy-formamidine as light brownish crystals (m.p. 70 ∿ 73°C).

9.0 g of the crystal obtained above, 5.3 g of potassium carbonate and 6.55 g of ethyl iodide were mixed with 50 ml of acetone and the mixture was heated under refluxing and stirring for 2 hours. The reaction mixture was cooled to the room temperature and then potassium iodide and potassium hydrogen carbonate were removed as crystals by filtration and they were washed with a small amount of acetone.

The filtrate were collected and acetone was distilled off from it. The crystals thus obtained were purified by recrystallization with methanol to yield 9.9 g of objective material of N-(3,5-diethyl-4-ethoxyphenyl)-N'-ethoxyformamidine as white crystals (m.p. 69 ∿ 71°C).

<u>Example 3</u> (Manufacturing Process B-a)

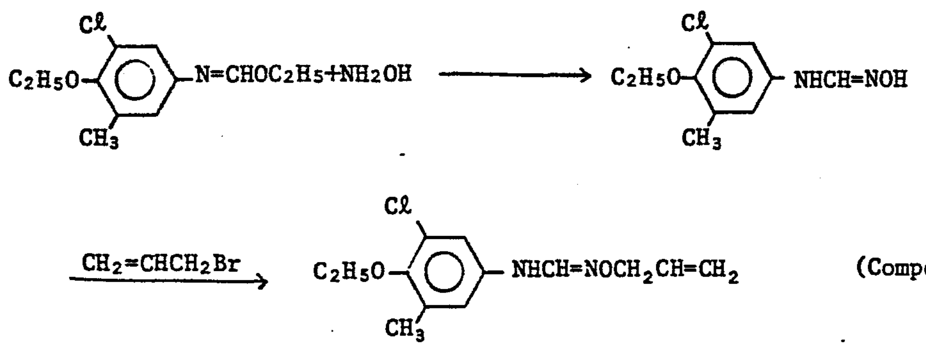


(Compound No. 106) :

1.1 g of hydroxyamine hydrochloride was dissolved in 10 ml of heated methanol and sodium methylate solution prepared by reacting 0.36 g of sodium metal and 10 ml of methanol at room temperature was dropped to the solution to neutralize it.

The resulting solution was cooled to about 5°C and sodium chloride was filtered off.

The filtrate was mixed with 3.1 g of crude intermediate material of ethyl N-(3-chloro-4-ethoxy-5-methylphenyl)-formimidate which was prepared from 2.4 g of 3-chloro-4-ethoxy-5-methylaniline by means of the similar process in Example 1 and the resulting mixture was stirred at room temperature for 2 hours.

Then, the methanol was distilled off and thereby, 2.9 g of crude intermediate material of N-(3-chloro-4-ethoxy-5-methylphenyl)-N'-hydroxy-formamidine was obtained.

The said intermediate material was dissolved in 20 ml of N,N-dimethylformamide and 1.6 g of triethylamine was added to the solution.

Further, the resulting solution was stirred at room temperature, and 1.9 g of allyl bromide was added into it and it was continuously stirred for 3 hours.

The reaction solution was poured into 100 ml of ice water and it was extracted with ethyl acetate.

The extracted solution was dried with anhydrous magnesium sulfate, and then, ethyl acetate was distilled off and the residue obtained was purified with silicagel column chromatography to yield 2.0 g of objective material of N-(3-chloro-4-ethoxy-5-methylphenyl)-N'-allyloxy-formamidine (m.p. 81 ∿ 83°C).

<u>Example 4</u> (Manufacturing Process B-a)

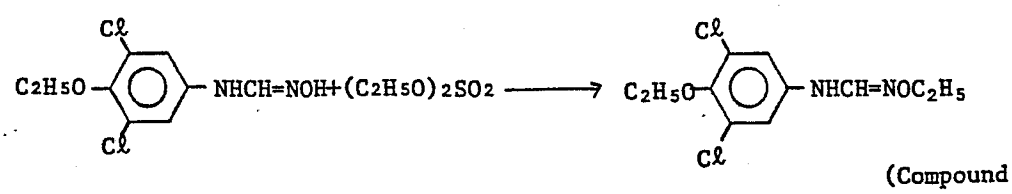

(Compound No. 7) :

To a mixture of 2.0 g of potasium carbonate and 3.5 g of N-(3,5-dichloro-4-ethoxyphenyl)-N'-hydroxy-formamidine in 25 ml N,N-dimethylformamide, 2.4 g of diethyl sulfate was added. The mixture was stirred at room temperature for 1 hour and at about 90°C for 2 hours. The mixture was then poured into ice water and was extracted with ethyl acetate. The extract dried over magnesium sulfate was evaporated under reduced pressure. The residue was purified by a silica gel column chromatography, and there was obtained 2.4 g of N-(3,5-dichloro-4-ethoxyphenyl)-N'-ethoxy-formamidine (m.p. 119.5 ∿ 120.5°C).

<u>Example 5</u> (Manufacturing Process B-b)

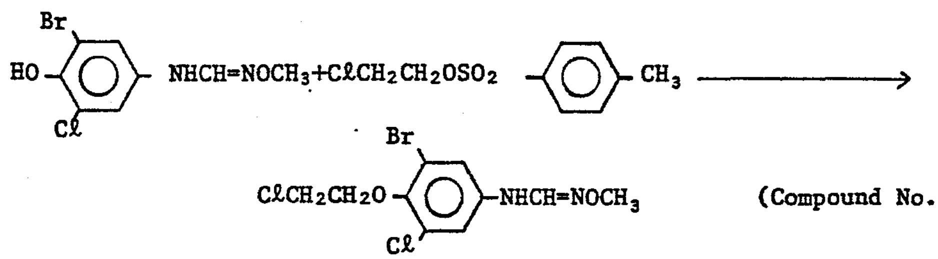

(Compound No. 78)

To a mixture of 0.45 g of sodium hydroxide and 3.0 g of N-(3-bromo-5-chloro-4-hydroxyphenyl)-N'-methoxy-formamidine in 20 ml N,N-dimethylformamide, 2.8 g of β-chloroethyl-p-toluenesulfonate was added. The mixture was stirred at room temperature for 5 hours. The mixture was then poured into ice water and was extracted with ethyl acetate. The extract dried over magnesium sulfate was evaporated under reduced pressure. The residue was purified by a silica gel column chromatography, and there was obtained 3.1 g of N-[3-bromo-5-chloro-4-(β-chloroethoxy)-phenyl]-N'-methoxy-formamidine (m.p. 112.5 ∿ 114.0°C).

Example 6

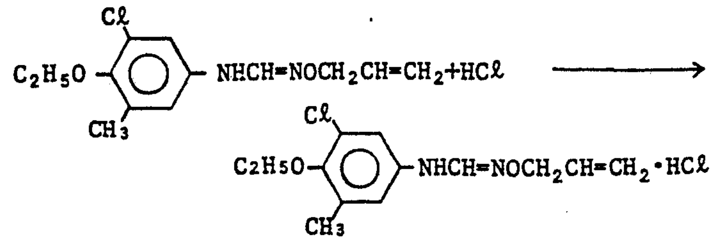

(Compound No. 434) :

1.0 g of N-(3-chloro-4-ethoxy-5-methylphenyl)-N'-allyloxy-formamidine was dissolved in 15 ml of chloroform and to the solution an excess amount of hydrochloric acid gas was introduced at room temperature under stirring, and then crystals were immediately precipitated.

Said separated crystals were gathered by filtration and they was dried in vacuum to yield 1.0 g of objective white crystals of N-(3-chloro-4-ethoxy-5-methylphenyl)-N'-allyloxy-formamidine hydrochloric acid. (m.p. 122 ∿ 123.5°C) dec.

Elementary Analysis:

| | C | H | N |
|---|---|---|---|
| Calculated Value | 51.16% | 5.94% | 9.18% |
| Measured Value | 51.04% | 6.01% | 9.31% |

Analysis for mol ratio of hydrochloric acid:

About 0.2 g of N-(3-chloro-4-ethoxy-5-methlphenyl)-N'-allyloxy-formamidine hydrochloric acid was precisely balanced and it was put in 30 ml of distilled water.

The distilled water was stirred at 50°C for 30 minutes and the hydrochloric acid salt was dissociated. The dissociated free material was separated and it was gathered by filtration and then washed with 70 ml of distilled water. The filtrate was collected in a 100 ml messflask and the distilled water is additionally filled so as it may reach to 100 ml of total volume and by means of shaking, a clear solution was obtained.

The water solution was collected with 25 ml whole pipet and it was neutralized by titrating with the aqueous solution containing 0.01 N of sodium hydroxide, and thereby, the quantity of hydrochloric acid was analyzed.

Said free material obtained by filtrating above, was dried, and then, it was identified by means of IR spectrum.

Sample quantity collected = Xg

Hydrochloric acid quantity in the collected sample measured by neutralizing titration = Y mol

$$\text{Mol ratio of HC}\ell\ (j) = \frac{X - Y \times 36.5}{258.5} \div Y$$

| X | Y | n |
|---|---|---|
| 0.20984 g | 0.0006702 mol | 1.03 |
| 0.21272 g | 0.0006569 mol | 1.07 |

Example 7

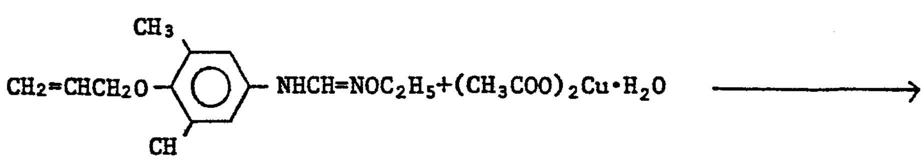

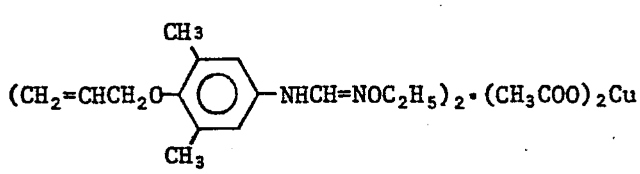

(Compound No. 402) :

1.7 g of N-(4-allyloxy-3,5-dimethylphenyl)-N'-ethoxy-formamidine was dissolved in 20 ml of acetone and to the solution was added 1.4 g of hydrated cupric acetate at room temperature under stirring. Imediately, its clear solution was obtained and it was continuously stirred at room temperature and crystals were precipitated after about 10 minutes.

The crystals were filtered and they were dried in a vacuum to yield 2.1 g of objective light green crystals of N-(4-allyloxy-3,5-dimethylphenyl)-N'-ethoxy-formamidine cupric acetate salt (m.p. 159 ∿ 160°C).

Elementary Analysis:

| | C | H | N |
|---|---|---|---|
| Calculated Value | 56.67% | 6.84% | 8.24% |
| Measured Value | 56.14% | 6.72% | 8.09% |

Analysis for mol ratio of cupric acetate:

About 0.16 g of N-(4-allyloxy-3,5-dimethylphenyl)-N'-ethoxy-formamidine cupric acetate salt was precisely balanced and it was put in 30 ml of distilled water and the resulting aqueous solution was stirred at 50°C for about 30 minutes and the salt was dissociated.

The dissociated free material was separated from the solution and it was gathered by filtration and sufficiently washed with 70 ml of distilled water.

The filtrate was collected in a 100 ml messflask and a supplementary distilled water was added so as it may be 100 ml of total volume and a clear-solution was obtained by means of shaking.

The aqueous solution was collected by 25 ml of whole pipet and it was chelatometric titrated by a conventional method using 0.01 N EDTA of standard solution, and the quantity of cupper was analyzed.

Free material obtained by the filtration above was dried and it was identified as the free material by IR spectrum.

Sample quantity collected = Xg

Copper quantity in the sample measured by chelating titration = Y mol

Mol ratio of cupric acetate (j) = $\frac{X - Y \times 181.5}{248} \div Y$

| X | Y | n |
|---|---|---|
| 0.16037 g | 0.0002311 mol | 2.07 |
| 0.16059 g | 0.0002260 mol | 2.13 |

Example 8

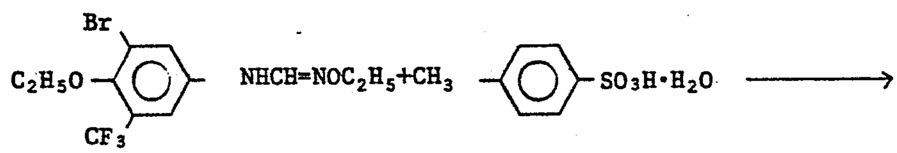

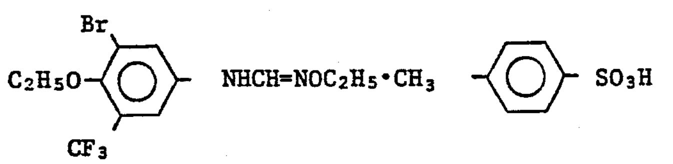

(Compound No. 437) :

1.6 g of N-(3-bromo-4-ethoxy-5-trifluoromethylphenyl)-N'-ethoxy-formamidine was dissolved in 30 ml of ethyl ether, and to the solution 0.86 g of hydrated p-toluenesulfonic acid was added at room temperature under stirring. Imediately, its clear solution was obtained and it was continuously stirred at room temperature and crystals were precipitated after about 3 minutes.

The crystals were gathered by filtration and they were dried in a vacuum to yield 2.3 g of white crystals of N-(3-bromo-4-ethoxy-5-trifluoromethylpenyl)-N'-ethoxy-formamidine p-toluenesulfonic acid salt (m.p. 146 ∿ 147.5°C).

Elementary Analysis:

| | C | H | N |
|---|---|---|---|
| Calculated Value | 56.67% | 6.84% | 8.24% |
| Measured Value | 56.14% | 6.72% | 8.09% |

Inclusive of the above, each compound within the scope of this invention which can be prepared in an analogeous manner is tabulated in Table 1.

Table 1

| Compound No. | 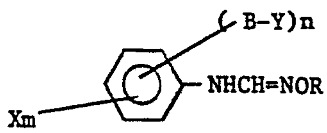 | | | Physical Properties [ ] m.p.°C |
|---|---|---|---|---|
| | $-(B-Y)n$ | Xm | R | |
| 1 | $4-OC_2H_5$ | - | $C_2H_5$ | $n_D^{27}$1.5550 |
| 2 | $4-OCH_2C\equiv CH$ | - | " | [61~63] |
| 3 | $4-OCF_3$ | - | " | $n_D^{27.5}$1.4920 |
| 4 | $4-OCH_2CH_2F$ | - | " | $n_D^{20.5}$1.5562 |
| 5 | $4-OCH_3$ | $3,5-C\ell_2$ | " | [119~120] |
| 6 | $4-OC_2H_5$ | " | $CH_3$ | [103~105] |
| 7 | " | " | $C_2H_5$ | [119.5~120.5] |
| 8 | " | " | $C_3H_7{}^n$ | [92~93] |
| 9 | " | " | $C_3H_7{}^i$ | [100~102] |
| 10 | " | " | $C_4H_9{}^n$ | [52~54] |
| 11 | " | " | $C_4H_9{}^i$ | [91~93] |
| 12 | " | " | $C_5H_{11}{}^n$ | [77~78] |
| 13 | " | " | $CH_2CH=CH_2$ | [102~103] |
| 14 | " | " | $CH_2C\equiv CH$ | [98~100] |
| 15 | " | " | $CH_2CH_2C\ell$ | [85~86.5] |
| 16 | " | " | $CH_2CH=CHC\ell$ (trans) | [79~81] |
| 17 | " | " | $CH_2CH=CHC\ell$ (cis) | [112~113] |
| 18 | " | " | $CH_2OCH_3$ | [90~94] |

| | | | | |
|---|---|---|---|---|
| 19 | 4-$OC_3H_7^n$ | 3,5-$Cl_2$ | $C_2H_5$ | [88.5~91.5] |
| 20 | 4-$OC_3H_7^i$ | " | " | [102~105] |
| 21 | 4-$OC_4H_9^n$ | " | " | [95~98] |
| 22 | 4-$OC_4H_9^i$ | " | " | [84~85.5] |
| 23 | 4-$OCH_2CH{=}CH_2$ | " | " | [101.5~103.5] |
| 24 | " | " | $C_3H_7^n$ | [105~106.5] |
| 25 | 4-$OCH_2CH{=}CHCH_3$ | " | $C_2H_5$ | [119~120.5] |
| 26 | 4-$OCH_2CH_2CH{=}CH_2$ | " | " | [86~87] |
| 27 | 4-$OCH_2C(CH_3){=}CH_2$ | " | " | [123~126] |
| 28 | 4-$OCH_2CH{=}C(CH_3)_2$ | " | " | [127.5~128] |
| 29 | 4-$OCH_2C{\equiv}CH$ | " | $CH_3$ | [131~133] |
| 30 | " | " | $C_2H_5$ | [136.5~137.5] |
| 31 | " | " | $C_3H_7^n$ | [126~128] |
| 32 | " | " | $CH_2CH{=}CH_2$ | [129~132] |
| 33 | " | " | $CH_2C{\equiv}CH$ | [131~132.5] |
| 34 | " | " | $CH_2CH_2F$ | [125~126] |
| 35 | " | " | $CH_2CF_3$ | [83~85] |
| 36 | " | " | $CH_2OCH_3$ | [145~149] |
| 37 | " | " | $CH_2OC_2H_5$ | [94~96] |
| 38 | " | " | $CH_2CH_2OC_2H_5$ | [101~102] |
| 39 | " | " | $CH_2SCH_3$ | [133~135] |
| 40 | " | " | $CH_2COOC_2H_5$ | [134~136] |
| 41 | 4-$OCH_2C{\equiv}CCH_3$ | " | $C_2H_5$ | [123.5~124] |
| 42 | 4-$OCH_2Cl$ | " | " | [130~132] |
| 43 | 4-$OCH_2F$ | " | " | [132~133] |
| 44 | 4-$OCHF_2$ | " | " | [152.5~153.5] |

| | | | | |
|---|---|---|---|---|
| 45 | 4-$OCH_2CH_2C\ell$ | 3,5-$C\ell_2$ | $C_2H_5$ | [93~95] |
| 46 | 4-$OCH_2CH_2F$ | " | $CH_3$ | [133~136.5] |
| 47 | " | " | $C_2H_5$ | [143~145.5] |
| 48 | " | " | $C_3H_7^i$ | [89~91] |
| 49 | " | " | $CH_2CH=CH_2$ | [132.5~133.5] |
| 50 | " | " | $CH_2C\equiv CH$ | [135~136] |
| 51 | " | " | $CH_2CH_2F$ | [143~144] |
| 52 | " | " | $CH_2OCH_3$ | [99.5~101.5] |
| 53 | " | " | $CH_2CH_2OC_2H_5$ | [80.5~82] |
| 54 | 4-$OCH_2CF_3$ | " | $C_2H_5$ | [144~145] |
| 55 | 4-$OCF_2CF_2H$ | " | " | [61~67] |
| 56 | 4-$OCH_2CH_2CH_2F$ | " | " | [77~78] |
| 57 | 4-$OCH_2C=CH_2$<br>\|<br>$C\ell$ | " | " | [130.5~136] |
| 58 | 4-$OCH_2CH=CHC\ell$ | " | " | [101~103] |
| 59 | 4-$OCH_2CH=CC\ell_2$ | " | " | [138.2~138.5] |
| 60 | 4-$OCH_2CH=CHF$ | " | " | [94~99] |
| 61 | 4-$OCHFCH=CH_2$ | " | " | [121~123] |
| 62 | 4-$OCH_2C\equiv CI$ | " | " | [149~154] |
| 63 | 4-$OCH_2CH_2OH$ | " | " | [110~114] |
| 64 | 4-$OCH_2OCH_3$ | " | " | [90~91.5] |
| 65 | 4-$OCH_2CH_2OC_2H_5$ | " | " | [65.3~66.1] |
| 66 | 4-$OCH_2CN$ | " | " | [146~149] |
| 67 | 4-$OCH_2COCH_3$ | " | " | [87~90] |
| 68 | 4-$OCH_2COOC_2H_5$ | " | " | [80~84] |
| 69 | 4-$OCHCOOC_2H_5$<br>\|<br>$CH_3$ | " | " | [51~53] |

(to be cont'd) 

| | | | | |
|---|---|---|---|---|
| 70 | 4-$OCH_2$- 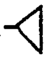 | 3,5-$Cl_2$ | $C_2H_5$ | [72∿75] |
| 71 | 4-$OCH_2$- 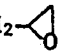 | " | " | [91∿94] |
| 72 | 4-$OCH_2C{\equiv}CCH_2OCH_3$ | " | " | [101.5∿102] |
| 73 | 4-$OCH_2CH_2OCOCH_3$ | " | " | [82∿83.5] |
| 74 | 4-$OCH_2CH_2SCH_3$ | " | " | [80∿81] |
| 75 | 4-$OCH_2CH_2SC_2H_5$ | " | " | [60∿61] |
| 76 | 4-$OCH_2CH_2NHCONH_2$ | " | " | [190∿193] |
| 77 | 4-$OC_2H_5$ | 3-Cl, 5-F | " | [71∿73] |
| 78 | 4-$OCH_2CH_2Cl$ | 3-Br, 5-Cl | $CH_3$ | [112.5∿114] |
| 79 | 4-$OC_2H_5$ | " | $C_2H_5$ | [124∿125] |
| 80 | " | " | $CH_2C{\equiv}CH$ | [114.5∿116] |
| 81 | " | 3,5-$Br_2$ | $C_2H_5$ | [122∿125] |
| 82 | " | 3,5-$I_2$ | " | [137.5∿138.5] |
| 83 | 4-$OCH_2CH{=}CH_2$ | 3-Br, 4-F | " | [64∿65] |
| 84 | " | 3-Br, 5-Cl | " | [118∿119] |
| 85 | " | 3,5-$Br_2$ | " | [125.5∿127.5] |
| 86 | 4-$OCH_2C{\equiv}CH$ | 3,5-$F_2$ | $CH_3$ | [100∿102] |
| 87 | " | " | $C_2H_5$ | [68∿71] |
| 88 | " | 3-Cl, 5-F | $CH_3$ | [84.5∿85.5] |
| 89 | " | " | $C_2H_5$ | [92∿93] |
| 90 | " | " | $CH_2CH{=}CH_2$ | [82.5∿83] |
| 91 | " | 3-Br, 5-F | $CH_3$ | [80∿82] |
| 92 | " | " | $C_2H_5$ | [99.5∿101] |
| 93 | " | " | $CH_2C{\equiv}CH$ | [98.5∿100] |
| 94 | " | " | $CH_2CH_2Cl$ | [66.5∿68] |
| 95 | " | 3-Br, 5-Cl | $CH_3$ | [149.5∿151] |

| | | | | |
|---|---|---|---|---|
| 96 | 4-$OCH_2C{\equiv}CH$ | 3-Br, 5-Cℓ | $C_2H_5$ | [141∿142] |
| 97 | " | " | $CH_2CH{=}CH_2$ | [139∿140] |
| 98 | " | 3,5-$Br_2$ | $C_2H_5$ | [142∿145] |
| 99 | 4-$OCH_2CH_2F$ | " | " | [161.5∿163.5] |
| 100 | 4-$OCH_2CF_3$ | 3-Br, 5-Cℓ | $CH_2CH{=}CH_2$ | [148.5∿150] |
| 101 | 4-$OCH_2CH_2Cℓ$ | 3-Br, 5-F | $C_2H_5$ | [78∿79] |
| 102 | 4-$OCH_2CN$ | " | " | [89∿91] |
| 103 | 4-$OCH_2C{\equiv}CH$ | 4-F, 5-$CH_2CH{=}CH_2$ | " | $n_D^{25}$1.5524 |
| 104 | 4-$OC_2H_5$ | 3-Cℓ, 5-$CH_3$ | $CH_3$ | [106∿108] |
| 105 | " | " | $C_2H_5$ | [97.5∿98.5] |
| 106 | " | " | $CH_2CH{=}CH_2$ | [81∿83] |
| 107 | " | " | $CH_2C{\equiv}CH$ | [100∿101] |
| 108 | " | " | $CH_2CH_2Cℓ$ | [58∿59] |
| 109 | 4-$OCH_2CH{=}CH_2$ | " | $C_2H_5$ | [65∿67] |
| 110 | 4-$OCH_2C{\equiv}CH$ | " | " | [75∿79] |
| 111 | 4-$OCH_2CF_3$ | " | " | [106∿107] |
| 112 | 4-$OCH_2CH_2Cℓ$ | " | " | $n_D^{17}$1.6615 |
| 113 | 4-$OCH_2CN$ | " | " | [84∿86] |
| 114 | 4-$OCH_2C{\equiv}CH$ | 3-Cℓ, 5-$C_2H_5$ | " | $n_D^{18.5}$1.5737 |
| 115 | " | 3-Cℓ, 5-$C_3H_7{}^n$ | " | $n_D^{20.5}$1.5679 |
| 116 | 4-$OC_2H_5$ | 3-Cℓ, 5-$C_3H_7{}^i$ | " | [67.5∿69.5] |
| 117 | 4-$OCH_2C{\equiv}CH$ | " | " | [53∿55] |
| 118 | " | 3-Cℓ, 5-$CH_2CH{=}CH_2$ | " | [52∿54] |
| 119 | " | 3-Cℓ, 5-$CF_3$ | $CH_3$ | [112∿115] |
| 120 | " | " | $C_2H_5$ | [112.5∿113.5] |
| 121 | 4-$OC_2H_5$ | 3-Br, 5-$C_2H_5$ | " | [82∿84] |
| 122 | 4-$OCH_2C{\equiv}CH$ | " | " | [54∿56] |

| | | | | |
|---|---|---|---|---|
| 123 | 4-$OC_2H_5$ | 3-Br, 5-$C_3H_7^n$ | $C_2H_5$ | [104.5~106.5] |
| 124 | 4-$OCH_2C{\equiv}CH$ | " | " | [53.5~54.5] |
| 125 | 4-$OC_2H_5$ | 3-Br, 5-$C_3H_7^i$ | " | [55~59] |
| 126 | 4-$OCH_2C{\equiv}CH$ | " | " | [66~68] |
| 127 | " | 3-Br, 5-$CH_2Cl$ | " | [98~99] |
| 128 | 4-$OC_2H_5$ | 3-Br, 5-$CF_3$ | " | [86.5~88.5] |
| 129 | 4-$OCH_2C{\equiv}CH$ | " | " | [123~124] |
| 130 | 4-$OCH_2CN$ | " | " | [118.5~119.5] |
| 131 | 4-$OC_2H_5$ | 3-Br, 5-$CH{=}CH_2$ | " | [77~78] |
| 132 | " | 3,5-$(CH_3)_2$ | " | [96~97] |
| 133 | " | " | $CH_2CH{=}CH_2$ | [81.5~82.5] |
| 134 | " | " | $CH_2C{\equiv}CH$ | [86.5~87.5] |
| 135 | " | 3,5-$(C_2H_5)_2$ | $C_2H_5$ | [69~71] |
| 136 | " | 3,5-$(C_3H_7^i)_2$ | " | [83~84] |
| 137 | " | 3,5-$(C_4H_9^t)_2$ | " | [81~83] |
| 138 | " | 3,5-$(CF_3)_2$ | " | [119~119.5] |
| 139 | 4-$OCH_2CH{=}CH_2$ | 3,5-$(CH_3)_2$ | " | [44~45] |
| 140 | 4-$OCH_2C{\equiv}CH$ | " | " | [42~49] |
| 141 | " | 3-$C_2H_5$, 5-$CH_3$ | " | $n_D^{20.5}$ 1.5620 |
| 142 | " | 3,5-$(C_2H_5)_2$ | " | $n_D^{21}$ 1.5535 |
| 143 | " | 3,5-$(C_3H_7^i)_2$ | " | $n_D^{23}$ 1.5470 |
| 144 | " | 3,5-$(C_4H_9^t)_2$ | " | $n_D^{21.5}$ 1.5424 |
| 145 | " | 3,5-$(CF_3)_2$ | $CH_3$ | [103~104] |
| 146 | " | " | $C_2H_5$ | [114~116] |
| 147 | 4-$OCH_2CH_2F$ | 3,5-$(CH_3)_2$ | " | [80~87] |
| 148 | " | 3,5-$(C_2H_5)_2$ | " | $n_D^{21}$ 1.5358 |
| 149 | 4-$OCH_2CH_2Cl$ | 3,5-$(CH_3)_2$ | " | $n_D^{14.5}$ 1.5508 |

(to be cont'd)



| | | | | |
|---|---|---|---|---|
| 150 | 4-$OCH_2C{\equiv}CH$ | 3-F, 5-$CH_2OCH_3$ | $C_2H_5$ | $n_D^{23.7}$1.5396 |
| 151 | " | 3-F, 5-$CH_2SCH_3$ | " | $n_D^{23.8}$1.5713 |
| 152 | " | 3-F, 5-$COCH_3$ | " | [95~97] |
| 153 | " | 3-F, 5-$COOCH_3$ | " | $n_D^{25}$1.5573 |
| 154 | " | 3-F, 5-$\overset{CH_3}{\overset{\vert}{C}}{=}NOC_2H_5$ | " | [69~72] |
| 155 | " | 3-Cℓ, 5-$CH_2OCH_3$ | " | [94~95] |
| 156 | " | 3-Cℓ, 5-$CH_2SCH_3$ | " | [57~60] |
| 157 | " | 3-Cℓ, 5-$COCH_3$ | " | [102~104] |
| 158 | " | 3-Cℓ, 5-$COOCH_3$ | " | [106~107.5] |
| 159 | " | 3-Br, 5-$CH_2OH$ | " | [100~102] |
| 160 | " | 3-Br, 5-$CH_2OCH_3$ | " | [89~92] |
| 161 | 4-$OC_2H_5$ | " | " | [62.5~63.5] |
| 162 | 4-$OCH_2CH{=}CH_2$ | " | " | [78.5~79.5] |
| 163 | 4-$OCH_2C{\equiv}CH$ | " | $CH_3$ | [86~88] |
| 164 | " | " | $CH_2CH{=}CH_2$ | [83.5~84.5] |
| 165 | " | 3-Br, 5-$CH_2OC_2H_5$ | " | [85~86] |
| 166 | " | 3-Br, 5-$CH{\genfrac{}{}{0pt}{}{OCH_3}{OCH_3}}$ | " | $n_D^{17.5}$1.5645 |
| 167 | " | 3-Br, 5-$CH_2OCH{\genfrac{}{}{0pt}{}{OC_2H_5}{OC_2H_5}}$ | " | $n_D^{21}$1.5432 |
| 168 | " | 3-Br, 5-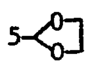 | " | [112~113] |
| 169 | " | 3-Br, 5-$CH_2N{\genfrac{}{}{0pt}{}{CH_3}{CH_3}}$ | " | $n_D^{25.5}$1.5817 |
| 170 | " | 3-Br, 5-$CHO$ | " | [137~139] |
| 171 | " | 3-Br, 5-$COOH$ | " | [163~168] |
| 172 | 4-$OC_2H_5$ | 3-Br, 5-$COOCH_3$ | $CH_2CH_2C\ell$ | [64~65] |
| 173 | 4-$OCH_2CH{=}CH_2$ | " | $C_2H_5$ | $n_D^{25.5}$1.5838 |

(to be cont'd)



| | | | | |
|---|---|---|---|---|
| 174 | 4-$OCH_2C{\equiv}CH$ | 3-Br, 5-$COOCH_3$ | $CH_3$ | [148∿149] |
| 175 | " | " | $C_2H_5$ | [114∿115] |
| 176 | " | " | $CH_2C{\equiv}CH$ | [103∿104] |
| 177 | 4-$OCH_2CH_2C\ell$ | " | $C_2H_5$ | [97∿99] |
| 178 | 4-$OCH_2C{\equiv}CH$ | 3-Br, 5-CN | " | [136∿137] |
| 179 | " | 3-Br, 5-$CONH_2$ | " | [185∿187] |
| 180 | " | 3-Br, 5-$CONHC_2H_5$ | " | [121∿123] |
| 181 | " | 3-Br, 5-CH=NOH | " | [137∿139] |
| 182 | " | 3-Br, 5-$CH{=}NOC_2H_5$ | " | [116∿118] |
| 183 | " | 3-Br, 5-CH=CHCN (trans) | " | [130∿132] |
| 184 | " | 3-Br, 5-CH=CHCN (cis) | " | [103∿105] |
| 185 | " | 3-Br, 5-$CH{=}CHCOOCH_3$ | " | [135∿137] |
| 186 | " | 3-Br, 5-$CH_2SCH_3$ | " | $n_D^{22.5}$1.6119 |
| 187 | " | 3-Br, 5-$CH_2\overset{\overset{O}{\uparrow}}{S}CH_3$ | " | $n_D^{24}$1.6135 |
| 188 | " | 3-$CH_3$, 5-$CH_2OCH_3$ | $CH_3$ | $n_D^{27.3}$1.5605 |
| 189 | " | " | $C_2H_5$ | $n_D^{26.4}$1.5577 |
| 190 | " | 3-$CH_3$, 5-$CH_2SCH_3$ | " | $n_D^{27.4}$1.5801 |
| 191 | " | 3-$CH_3$, 5-$COOCH_3$ | " | [59 61] |
| 192 | " | 3-$C_3H_7^i$, 5-$CH_2OCH_3$ | " | $n_D^{26.5}$1.5424 |
| 193 | " | 3-$C_3H_7^i$, 5-$CH_2SCH_3$ | " | $n_D^{26.5}$1.5655 |
| 194 | " | 3-$C_3H_7^i$, 5-$COOCH_3$ | " | $n_D^{27.5}$1.5466 |
| 195 | " | 3-$CF_3$, 5-$CH_2OCH_3$ | " | [62∿64.5] |
| 196 | " | 3-$CF_3$, 5-$CH_2SCH_3$ | " | |
| 197 | " | 3-$CF_3$, 5-$COOCH_3$ | " | [79∿80] |
| 198 | 4-$OC_2H_5$ | 3-$CH_3$ | " | $n_D^{26}$1.5438 |

| | | | | |
|---|---|---|---|---|
| 199 | 4-$OC_2H_5$ | 3-$C_2H_5$ | $C_2H_5$ | [43∿45] |
| 200 | " | 3-$C_3H_7^n$ | " | $n_D^{18}$1.5380 |
| 201 | " | 3-$C_3H_7^i$ | " | $n_D^{18}$1.5375 |
| 202 | " | 3-$CF_3$ | " | [91.5∿93.5] |
| 203 | " | 2-$CH_3$ | " | $n_D^{22}$1.5464 |
| 204 | 4-$OCH_2C{\equiv}CH$ | 3-F | " | $n_D^{26}$1.5477 |
| 205 | 4-$OC_2H_5$ | " | " | $n_D^{19.5}$1.5483 |
| 206 | 4-$OCH_2C{\equiv}CH$ | 3-Cℓ | " | [65.5∿68] |
| 207 | " | 3-Br | " | [74∿76] |
| 208 | " | 3-$CH_3$ | " | $n_D^{16.5}$1.5674 |
| 209 | " | 3-$CF_3$ | " | $n_D^{17}$1.5197 |
| 210 | " | 3-COOH | " | [176∿181] |
| 211 | " | 3-$COOCH_3$ | " | [105∿106] |
| 212 | " | 3-$CONH_2$ | " | [182∿185] |
| 213 | " | 3-$CONHC_2H_5$ | " | [94∿95] |
| 214 | " | 3-$CH{=}NOC_2H_5$ | " | $n_D^{24}$1.5615 |
| 215 | " | 3-$CH_2OH$ | " | [75∿78] |
| 216 | 4-$OCH_2CH_2F$ | 3-Cℓ | " | [60.5∿62] |
| 217 | " | 3-$CH_3$ | " | [60∿62] |
| 218 | " | 2-$CH_3$ | " | $n_D^{21.5}$1.5440 |
| 219 | 4-$OCF_3$ | 3-Cℓ | " | [51∿55] |
| 220 | 4-$OCH_2CF_3$ | 3-$CF_3$ | " | [101∿102] |
| 221 | 4-$OCH_2CN$ | 3-$CH_3$ | " | $n_D^{17.5}$1.5690 |
| 222 | 4-$OC_2H_5$ | 2,3-$(CH_3)_2$ | " | [56∿57] |
| 223 | " | 2,5-$(CH_3)_2$ | " | [72∿73] |
| 224 | 4-$OCH_2C{\equiv}CH$ | 2,3-$C\ell_2$ | " | [104∿105] |
| 225 | " | 2,6-$C\ell_2$ | " | [62∿66] |

(to be cont'd)



| | | | | |
|---|---|---|---|---|
| 226 | 4-$OCH_2C{\equiv}CH$ | 2,6-$(CH_3)_2$ | $C_2H_5$ | [78∿80] |
| 227 | 4-$OCH_2CH_2F$ | 2,3-$(CH_3)_2$ | " | [69∿72] |
| 228 | " | 2,5-$(CH_3)_2$ | " | [86.5∿88] |
| 229 | 4-$OC_2H_5$ | 2-F, 3,5-$C\ell_2$ | " | [76∿80] |
| 230 | " | 2-$C\ell$, 3,5-$(CH_3)_2$ | " | [70∿72] |
| 231 | " | 2,3,5-$(CH_3)_3$ | " | [46∿51] |
| 232 | 4-$OCH_2C{\equiv}CH$ | 2,3,5-$C\ell_3$ | " | [155∿158] |
| 233 | " | 2-$CH_3$, 3,5-$C\ell_2$ | " | [142∿143] |
| 234 | " | 2,3,6-$(CH_3)_3$ | " | [75∿76] |
| 235 | 4-$OC_2H_5$ | 2,3,5,6-$(CH_3)_4$ | " | [56∿59] |
| 236 | 4-$OCH_2C{\equiv}CH$ | 2,3,5,6-$C\ell_4$ | " | [114∿117] |
| 237 | 3,4-$(OCH_3)_2$ | - | " | $n_D^{27}$1.5642 |
| 238 | 3,4-$(OC_2H_5)_2$ | - | " | $n_D^{28}$1.5483 |
| 239 | 3,4-$(OC_3H_7^n)_2$ | - | " | [48∿50] |
| 240 | 3,4-$(OC_3H_7^i)_2$ | - | " | $n_D^{21}$1.5308 |
| 241 | 3,4-$(OCH_2CH{=}CH_2)_2$ | - | " | $n_D^{22}$1.5590 |
| 242 | 3-$OC_2H_5$, 4-$OCH_2C{\equiv}CH$ | - | " | [84∿85] |
| 243 | 3,4-$(OCH_2C{\equiv}CH)_2$ | - | " | [79∿80] |
| 244 | 3-$OCH_3$, 4-$OC_2H_5$ | 5-$C\ell$ | " | [121∿122] |
| 245 | " | 5-Br | " | [131∿132.5] |
| 246 | 3,4-$(OC_2H_5)_2$ | 5-$C\ell$ | " | [110∿111] |
| 247 | 3-$OCH_2C{\equiv}CH$, 4-$OC_2H_5$ | " | $CH_3$ | [86∿87] |
| 248 | " | " | $C_2H_5$ | [58∿59] |
| 249 | 3-$OCH_3$, 4-$OC_2H_5$ | 5-$CH_3$ | " | [106∿107] |
| 250 | 3,4-$(OC_2H_5)_2$ | " | " | [89∿91] |
| 251 | 3,4-$(OCH_2CH{=}CH_2)_2$ | 5-Br | " | [52∿53.5] |
| 252 | " | 5-$CH_3$ | " | $n_D^{16.5}$1.5560 |

| | | | | |
|---|---|---|---|---|
| 253 | 3-$OCH_3$, 4-$OCH_2C{\equiv}CH$ | 5-Cℓ | $C_2H_5$ | [78∿79] |
| 254 | " | 5-Br | " | [109∿110] |
| 255 | 3-$OC_2H_5$, 4-$OCH_2C{\equiv}CH$ | 5-Cℓ | " | [87∿88.5] |
| 256 | 3-$OC_3H_7{}^n$, 4-$OCH_2C{\equiv}CH$ | " | " | [81∿82] |
| 257 | 3-$OCH_2CH{=}CH_2$, 4-$OCH_2C{\equiv}CH$ | " | $CH_3$ | [57∿58.5] |
| 258 | " | " | $C_2H_5$ | [70∿71] |
| 259 | 3,4-$(OCH_2C{\equiv}CH)_2$ | 5-F | $CH_3$ | [86∿88] |
| 260 | " | " | $C_2H_5$ | $n_D^{20}$1.5491 |
| 261 | " | " | $CH_2C{\equiv}CH$ | [97∿98.5] |
| 262 | " | 5-Cℓ | $CH_3$ | [71∿73] |
| 263 | " | " | $C_2H_5$ | [78∿79] |
| 264 | " | " | $CH_2CH{=}CH_2$ | [86∿87.5] |
| 265 | " | " | $CH_2C{\equiv}CH$ | [68.5∿70.5] |
| 266 | " | " | $CH_2OCH_3$ | [84∿86] |
| 267 | " | 5-Br | $C_2H_5$ | [84∿85] |
| 268 | 3-$OCH_3$, 4-$OCH_2C{\equiv}CH$ | 5-$CH_3$ | " | [82.5∿83.5] |
| 269 | 3-$OC_2H_5$, 4-$OCH_2C{\equiv}CH$ | " | " | [53∿56] |
| 270 | 3,4-$(OCH_2C{\equiv}CH)_2$ | " | " | $n_D^{18}$1.5600 |
| 271 | " | 5-COOH | $C_2H_5$ | [100∿103] |
| 272 | " | 5-$COOCH_3$ | " | [64∿66] |
| 273 | " | 5-$COOC_2H_5$ | " | [80∿81] |
| 274 | " | 5-$COOCH_2CH{=}CH_2$ | " | [79∿80] |
| 275 | " | 5-$COOCH_2C{\equiv}CH$ | " | $n_D^{24.5}$1.5704 |
| 276 | " | 5-$CONH_2$ | " | [160∿161] |
| 277 | " | 5-$CONHC_2H_5$ | " | [74∿77] |
| 278 | 2,4-$(OCH_2C{\equiv}CH)_2$ | - | $C_2H_5$ | [49.5∿51.5] |
| 279 | 2,4-$(OC_2H_5)_2$ | 3,5-$Cℓ_2$ | " | [101∿102] |

| | | | | |
|---|---|---|---|---|
| 280 | 2,4-$(OCH_2C{\equiv}CH)_2$ | 3,5-$C\ell_2$ | $C_2H_5$ | [153∿157] |
| 281 | 3,5-$(OCH_3)_2$, 4-$OC_2H_5$ | - | " | [93∿95] |
| 282 | 5-$OCH_3$, 3,4-$(OC_2H_5)_2$ | - | " | [75∿76.5] |
| 283 | 3,4,5-$(OC_2H_5)_3$ | - | " | $n_D^{14.5}$1.5521 |
| 284 | 5-$OCH_3$, 3,4-$(OCH_2CH{=}CH_2)_2$ | - | " | $n_D^{22}$1.5515 |
| 285 | 3,5-$(OCH_3)_2$, 4-$OCH_2C{\equiv}CH$ | - | " | $n_D^{18}$1.5560 |
| 286 | 5-$OCH_3$, 3,4-$(OCH_2C{\equiv}CH)_2$ | - | " | $n_D^{17.5}$1.5659 |
| 287 | 3,4,5-$(OCH_2C{\equiv}CH)_3$ | - | " | [63∿65] |
| 288 | 2-$OCH_3$ | - | " | $n_D^{24}$1.5607 |
| 289 | 2-$OC_2H_5$ | - | " | $n_D^{27.5}$1.5425 |
| 290 | 2-$OC_3H_7{}^n$ | - | " | $n_D^{26}$1.5414 |
| 291 | 2-$OC_3H_7{}^i$ | - | " | $n_D^{30}$1.5259 |
| 292 | 2-$OCH_2CH{=}CH_2$ | - | " | $n_D^{26}$1.5491 |
| 293 | 2-$OCH_2C{\equiv}CH$ | - | " | $n_D^{26.5}$1.5613 |
| 294 | 2-$OCH_2CH_2F$ | - | " | [46.5∿48.5] |
| 295 | 2-$OC_2H_5$ | 5-$C\ell$ | " | [77∿79] |
| 296 | " | 3,5-$C\ell_2$ | " | [111.9∿112.3] |
| 297 | " | 3,5-$(CH_3)_2$ | " | [65∿68] |
| 298 | " | 3,5-$C\ell_2$, 4-F | " | [111∿113] |
| 299 | " | 3,5-$C\ell_2$, 4-$CH_3$ | " | [113∿114] |
| 300 | 2-$OC_3H_7{}^n$ | 3,5-$C\ell_2$ | " | [77∿78] |
| 301 | 2-$OCH_2C{\equiv}CH$ | " | " | [142.5∿145.5] |
| 302 | " | 4,6-$C\ell_2$ | " | [58∿63] |
| 303 | " | 3,5-$C\ell_2$, 4-F | " | [139∿140] |
| 304 | " | 3,5-$(CH_3)_2$ | " | [108∿110] |
| 305 | 2-$OCH_2CH_2F$ | " | " | [100∿101] |
| 306 | 3-$OC_2H_5$ | - | " | $n_D^{23.5}$1.5590 |

(to be cont'd)



| | | | | |
|---|---|---|---|---|
| 307 | 3-$OC_2H_5$ | 5-F | $C_2H_5$ | $n_D^{18.5}$1.5467 |
| 308 | " | 4-Cℓ, 6-F | " | [59.5∿60.5] |
| 309 | 3-$OCH_2CH$=$CH_2$ | " | " | [61∿63.5] |
| 310 | 3-$OCH_2C{\equiv}CH$ | " | " | [100∿101.5] |
| 311 | 3-$OCH_2CH_2F$ | - | " | $n_D^{20}$1.5598 |
| 312 | 2,3,4-$(OCH_3)_3$ | - | " | [68∿70] |
| 313 | 2,5-$(OC_2H_5)_2$ | - | " | $n_D^{24}$1.5339 |
| 314 | 2,6-$(OCH_3)_2$ | - | " | $n_D^{27.5}$1.5518 |
| 315 | 3,5-$(OCH_3)_2$ | - | " | $n_D^{25}$1.5739 |
| 316 | - | - | " | $n_D^{23.5}$1.5670 |
| 317 | - | 2-Cℓ | " | $n_D^{23}$1.5680 |
| 318 | - | 3-Cℓ | " | $n_D^{25}$1.5754 |
| 319 | - | 4-Cℓ | " | [94.5∿95.5] |
| 320 | - | 2,3-$C\ell_2$ | " | [84∿85] |
| 321 | - | 2,4-$C\ell_2$ | " | [85.5∿86.5] |
| 322 | - | 2,5-$C\ell_2$ | " | [109∿110.5] |
| 323 | - | 2,6-$C\ell_2$ | " | $n_D^{25}$1.5774 |
| 324 | - | 3,4-$C\ell_2$ | " | [105∿106] |
| 325 | - | 3,5-$C\ell_2$ | $CH_3$ | [110∿112] |
| 326 | - | " | $C_2H_5$ | [103∿106] |
| 327 | - | " | $C_3H_7^n$ | [82.5∿84.5] |
| 328 | - | " | $C_3H_7^i$ | [91∿95] |
| 329 | - | " | $C_4H_9^n$ | [66.5∿67.5] |
| 330 | - | " | $C_{12}H_{25}^n$ | [68∿72] |
| 331 | - | " | $CH_2CH$=$CH_2$ | [89∿92] |
| 332 | - | " | $CH_2C{\equiv}CH$ | $n_D^{21.5}$1.5959 |
| 333 | - | 3,5-$Br_2$ | $C_2H_5$ | [139∿141] |

(to be cont'd)



| | | | | |
|---|---|---|---|---|
| 334 | - | 3,5-$(CH_3)_2$ | $C_2H_5$ | $n_D^{25}$1.5590 |
| 335 | - | 3,5-$(CF_3)_2$ | " | [99∿100.5] |
| 336 | - | 3-$CF_3$ | " | $n_D^{23}$1.5111 |
| 337 | - | 3-Cℓ, 2-F | " | [50∿51] |
| 338 | - | 2-$CH_3$, 3-$NO_2$ | " | [58∿59] |
| 339 | - | 4-Cℓ, 2-$CH_3$ | " | [35∿36] |
| 340 | - | 2,4,5-$F_3$ | " | [78∿79] |
| 341 | - | 2,4,6-$F_3$ | " | $n_D^{18}$1.5033 |
| 342 | - | 2,3,4-$Cℓ_3$ | " | [96∿99] |
| 343 | - | 2,4,5-$Cℓ_3$ | " | [158∿159] |
| 344 | - | 2,4,6-$Cℓ_3$ | $CH_3$ | [92∿96] |
| 345 | - | " | $C_2H_5$ | [80∿82] |
| 346 | - | " | $C_3H_7^n$ | [33∿37] |
| 347 | - | 3,4,5-$Cℓ_3$ | $C_2H_5$ | [170∿171] |
| 348 | - | 3,5-$Cℓ_2$, 4-F | " | [108.5∿111] |
| 349 | - | 3,5-$Cℓ_2$, 4-$CH_3$ | " | [134∿138] |
| 350 | - | 3,5-$Cℓ_2$, 4-$C_2H_5$ | " | [137∿139.5] |
| 351 | - | 3,5-$Cℓ_2$, 4-$C_4H_9^n$ | " | [109∿110.5] |
| 352 | - | 3,5-$Cℓ_2$, 4-$CH_2OCH_3$ | " | [129∿130] |
| 353 | - | 3,5-$Cℓ_2$, 4-$CH_2OC_2H_5$ | " | [120∿124] |
| 354 | - | 3,5-$Cℓ_2$, 4-CN | " | [255∿258] |
| 355 | 4-SH | 3,5-$Cℓ_2$ | " | [167∿169] |
| 356 | 4-$SC_2H_5$ | 3,5-$Cℓ_2$ | " | [108.5∿109.5] |
| 357 | 4-$SCH_2C≡CH$ | 3,5-$Cℓ_2$ | " | [136∿137.5] |
| 358 | 4-$SCH_2CH_2F$ | 3,5-$Cℓ_2$ | " | [144.5∿146] |
| 359 | 4-$\overset{O}{\uparrow}{S}C_2H_5$ | 3,5-$Cℓ_2$ | " | [138∿142] |

(to be cont'd)



| | | | | |
|---|---|---|---|---|
| 360 | 4-$SO_2C_2H_5$ | 3,5-$Cl_2$ | $C_2H_5$ | [128∿133] |
| 361 | 4-$NHC_2H_5$ | 3,5-$Cl_2$ | " | $n_D^{22.5}$1.6045 |
| 362 | 4-$NHCH_2CH_2F$ | 3,5-$Cl_2$ | " | [74∿77] |
| 363 | 4-$N(C_2H_5)_2$ | 3,5-$Cl_2$ | " | [83∿85] |
| 364 | - | 3,5-$Cl_2$, 4-I | " | [193∿195] |
| 365 | - | 2,3,5,6-$F_4$ | " | $n_D^{18.5}$1.4892 |
| 366 | - | 2,3,5,6-$Cl_4$ | " | [80∿83] |
| 367 | - | 2,3,5-$Cl_3$, 4-$C_4H_9^n$ | " | [127∿131] |
| 368 | - | 2,4-$Br_2$, 3,5-$(CH_3)_2$ | " | [139∿140] |
| 369 | 3,4-(-$OCH_2O$-) | | " | $n_D^{28}$1.5780 |
| 370 | $OC_2H_5$<br>\|<br>3,4-(-OCHO-) | | " | $n_D^{26.5}$1.5468 |
| 371 | 4,5-(-$OCH_2OCH_2$-) | 3-Cl | " | [109∿111] |
| 372 | " | 3-$CH_3$ | " | [77∿78] |
| 373 | O<br>‖<br>4,5-(-$OCH_2OC$-) | 3-Cl, | " | [213∿215] |
| 374 | " | 3-$CH_3$ | " | [173∿175] |
| 375 | 4-$OC_2H_5$ | 3-Br, 5-$CH_2SO_2CH_3$ | " | [143 147] |
| 376 | " | 3-Br, 5-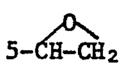 | " | $n_D^{26.5}$1.5723 |
| 377 | 4-$OCH_2C{\equiv}CH$ | 3,5-$Cl_2$ | $CH_2CN$ | [114∿119] |
| 378 | " | 3-Cl, 5-$CH_2OCH_3$ | $CH_3$ | [57∿59] |
| 379 | 4-$OCH_2CN$ | 3-Br, 5-$CH_2OCH_3$ | $C_2H_5$ | [135∿137] |
| 380 | 3,4-($OCH_2C{\equiv}CH$) | 5-$CH_2OCH_3$ | " | $n_D^{27.5}$1.5520 |
| 381 | " | 5-$CH_2OCH_2CH{=}CH_2$ | " | $n_D^{28}$1.5589 |
| 382 | " | 5-$CH_2OCH_2C{\equiv}CH$ | " | |
| 383 | " | 5-$CH_2CN$ | " | [122∿124] |

| | | | | |
|---|---|---|---|---|
| 384 | " | 5-CH=$CH_2$ | " | $n_D^{26.5}$1.5845 |
| 385 | " | 5-$CH_2SCH_3$ | " | $n_D^{27}$1.5872 |
| 386 | " | 5-CH=$NOCH_3$ | " | [86~88] |
| 387 | " | 5-CH=NOH | " | [131~133] |
| 388 | " | 5-CN | " | [120.5~122] |
| 389 | " | 2-$COOCH_3$ | " | [67~69] |
| 390 | 3-OCH=C=$CH_2$, 4-$OC_2H_5$ | 5-Cℓ | " | [54~55] |
| 391 | 4-$OCH_2CH_2C$ℓ | 2,5-$(CH_3)_2$ | " | [74~75] |
| 392 | 2,4,5-$(OCH_3)_3$ | - | " | [40~42] |
| 393 | - | 3,5-Cℓ$_2$ | $CH_2OCH_3$ | [103~104] |
| 394 | - | " | $CH_3$ <br> \| <br> $CHCOOC_2H_5$ | [85~86] |
| 395 | - | 2,6-$(CH_3)_2$ | $C_2H_5$ | [47.5~48.5] |
| 396 | - | 3,5-$(COOC_3H_7{}^i)$ | " | $n_D^{21}$1.5248 |
| 397 | - | 3,5-Cℓ$_2$, 2,4-$F_2$ | " | [122~122.5] |
| 397 -1 | - | 2-$COOCH_3$ | " | [46~47] |
| 397 -2 | 3,4-$(OCH_2C{\equiv}CH)_2$ | 5-$CH_2OH$ | " | $n_D^{27}$1.5755 |
| 397 -3 | 3-$OCH_2C{\equiv}CH$, 4-$OCH_3$ | 5-$CH_2OCH_3$ | " | $n_D^{27}$1.5534 |
| 397 -4 | 3,4-$(OCH_2C{\equiv}CH)_2$ | O <br> ‖ <br> 5-$CH_2OCOCH_2C{\equiv}CH$ | " | $n_D^{27}$1.5438 |
| 397 -5 | - | 2,3,5-Cℓ$_3$ | " | [110~111] |
| 397 -6 | 3,4-$(OC_2H_5)_2$ | - | $C_3H_7{}^i$ | $n_D^{28.5}$1.5372 |
| 397 -7 | 3,4-$(OCH_2C{\equiv}CH)_2$ | - | " | $n_D^{28}$1.5600 |
| 397 -8 | 5-$OCH_2C{\equiv}CH$ | 4-Cℓ, 2-F | $CH_3$ | [121~122] |
| 397 -9 | " | 2,4-Cℓ$_2$ | $C_2H_5$ | [144~145] |

| Compound No. | 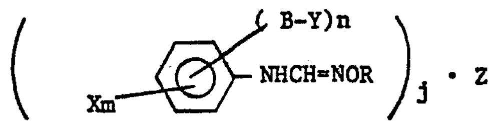 | | | | | physical properties [ ] m.p.°C |
|---|---|---|---|---|---|---|
| | ⟵B-Y)n | Xm | R | Z | j | |
| 398 | 4-$OC_2H_5$ | 3-$CH_3$ | $C_2H_5$ | $(COOH)_2$ | 1 | [104~108] dec. |
| 399 | " | 3-$C_2H_5$ | " | " | " | [100~103] dec. |
| 400 | " | 3,5-$(CH_3)_2$ | " | HCℓ | " | [128.5~130.5] dec. |
| 401 | " | 3,5-$(C_3H_7^i)_2$ | " | HBr | " | [167~168] dec. |
| 402 | 4-$OCH_2CH=CH_2$ | 3,5-$(CH_3)_2$ | " | $(CH_3COO)_2Cu$ | 2 | [159~160] |
| 403 | " | " | " | 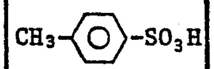 | 1 | [72~74] |
| 404 | " | " | " | $(COOH)_2$ | " | [107~109] dec. |
| 405 | " | " | " | HCℓ | " | [163~166] dec. |
| 406 | " | " | " | $SiCℓ_4$ | 4 | [166~169] dec. |
| 407 | 4-$OC_2H_5$ | 3,5-$Cℓ_2$ | " | HCℓ | 1 | [123~126] dec. |
| 408 | " | " | " | $CuCℓ_2$ | 2 | [203~206] |
| 409 | " | " | " | $ZnCℓ_2$ | " | [225~226] |
| 410 | " | " | " | $H_3PO_4$ | 3 | [132~134] dec. |
| 411 | " | " | " | $(COOH)_2$ | 1 | [110~115] dec. |
| 412 | " | " | " | $MnCℓ_2$ | 2 | [126~130] |
| 413 | " | " | " | $AℓCℓ_3$ | 3 | [159~162] |
| 414 | 4-$OCH_2CH=CH_2$ | " | " | HCℓ | 1 | [138~140] dec. |

| | | | | | | |
|---|---|---|---|---|---|---|
| 415 | 4-$OCH_2CH=CH_2$ | 3,5-$Cl_2$ | $C_2H_5$ | $CuCl_2$ | 2 | [160~161] |
| 416 | 4-$OCH_2C\equiv CH$ | " | " | $HCl$ | 1 | [139~143] dec. |
| 417 | " | " | " | $CuCl_2$ | 2 | [170~173] |
| 418 | " | " | " | $(CH_3COO)_2Cu$ | " | >300 |
| 419 | " | " | " | $HBr$ | 1 | [181~183] dec. |
| 420 | " | " | " | $AlCl_3$ | 3 | [144~145.5] |
| 421 | " | " | " | 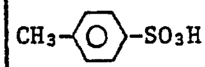 | 1 | [86~88] |
| 422 | " | " | " | $SiCl_4$ | 4 | [131~133] |
| 423 | " | " | $CH_2CH=CH_2$ | $HCl$ | 1 | [130~132.5] dec. |
| 424 | " | " | " | $CuCl_2$ | 2 | [144.5~145.5] |
| 425 | " | 3,5-$Br_2$ | $C_2H_5$ | $HCl$ | 1 | [147.5~148.5] dec. |
| 426 | " | " | " | $CuCl_2$ | 2 | [>300] |
| 427 | 4-$OCH_2CH_2Cl$ | 3,5-$Cl_2$ | $C_2H_5$ | " | " | [177~178] |
| 428 | " | " | " | 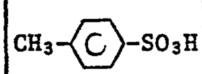 | 1 | [117~120] |
| 429 | 4-$OCH_2CF_3$ | " | " | $HCl$ | " | [138~139] dec. |
| 430 | " | " | " | $CuCl_2$ | 2 | [180~183] |
| 431 | 4-$OCH_2CH=CH_2$ | 3-Br, 5-F | " | $(CH_3COO)_2Cu$ | " | [178~180] |
| 432 | 4-$OCH_2C\equiv CH$ | " | " | $HCl$ | 1 | [161~164] dec. |
| 433 | 4-$OCH_2CH_2Cl$ | " | " | $(COOH)_2$ | " | [100~104] dec. |
| 434 | 4-$OC_2H_5$ | 3-Cl, 5-$CH_3$ | $CH_2CH=CH_2$ | $HCl$ | " | [122~123.5] dec. |
| 435 | " | " | $C_2H_5$ | $(COOH)_2$ | " | [124~127] dec. |
| 436 | " | 3-Br, 5-$CF_3$ | " | $H_3PO_4$ | 3 | [107~111] dec. |

| | | | | | | |
|---|---|---|---|---|---|---|
| 437 | 4-$OC_2H_5$ | 3-Br, 5-$CF_3$ | $C_2H_5$ | 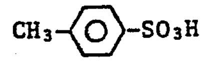 | 1 | [146~147.5] |
| 438 | " | " | " | $SiCl_4$ | 4 | [121~125] dec. |
| 439 | " | " | " | $(COOH)_2$ | 1 | [130~132] dec. |
| 440 | 4,5-$(OC_2H_5)_2$ | 3-Cl | " | 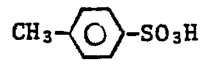 | " | [75 ~ 78] dec. |
| 441 | 4-$OCH_2C{\equiv}CH$, 5-$OCH_3$ | 3-Br | " | $(CH_3COO)_2Cu$ | 2 | [ 153-157]dec. |
| 442 | 4,5-$(OCH_2CH{=}CH_2)_2$ | " | " | 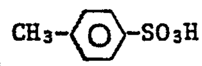 | 1 | [72~75] |
| 443 | 4,5-$(OCH_2C{\equiv}CH)_2$ | " | " | " | " | [35~38] |
| 444 | 3,4-$(OC_2H_5)_2$, 5-$OCH_3$ | - | " | $H_3PO_4$ | 3 | [90~91] dec. |
| 445 | " | - | " | 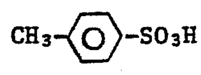 | 1 | [75~77] |
| 446 | 3,5-$(OCH_3)_2$, 4-$OC_2H_5$ | - | " | " | " | [74~77] |
| 447 | - | 3,5-$Cl_2$ | " | HCl | " | [109~111.5] dec. |
| 448 | - | " | " | $(COOH)_2$ | " | [98~101] dec. |
| 449 | - | " | " | $H_3PO_4$ | 3 | [133~134] dec. |
| 450 | - | 2,3-$Cl_2$ | " | HCl | 1 | [85~87] dec. |
| 451 | - | 2,4-$Cl_2$ | " | " | " | [81~83] dec. |
| 452 | - | 3,5-$Cl_2$ | $CH_2CH{=}CH_2$ | " | " | [209~213] dec. |
| 453 | - | " | " | $(COOH)_2$ | " | [184~185] dec. |
| 454 | 4-$OCH_2C{\equiv}CH$ | " | $C_2H_5$ | " | " | [128~130] dec. |
| 455 | - | 2,3-$Cl_2$ | " | $CuCl_2$ | 2 | [196~197] |

(to be cont'd)



| | | | | | | |
|---|---|---|---|---|---|---|
| 456 | - | 2,4-$C\ell_2$ | $C_2H_5$ | $CuC\ell_2$ | 2 | [217∿217.5] |
| 457 | - | 3,5-$C\ell_2$ | " | " | " | [181.5∿182] |
| 458 | - | " | " | $A\ell C\ell_3$ | 3 | [107.5∿108.5] |
| 459 | 4-$OCH_2C\equiv CH$ | 3-$C_3H_7^i$, 5-$COOCH_3$ | " | $CH_3$- 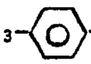 -$SO_3H$ | 1 | [67∿72] |
| 460 | 4-$OCH_2CH{=}CH_2$ | 3-Br, 5-$CH_2OCH_3$ | " | $HC\ell$ | " | [142∿146] dec. |
| 461 | 4-$OCH_2C\equiv CH$ | 3-Br, 5-$COOCH_3$ | $CH_3$ | $(CH_3COO)_2Cu$ | 2 | [180∿183] dec. |

As already mentioned, the compound having the formula [I] and Mixed Chemicals exhibits an outstanding fungicidal, insecticidal and/or acaricidal fficacy and the compositions containing said compound or a kind of Mixed Chemicals as active ingredient(s) may be formulated by mixing suitable carrieres and/or additives generally used in agricultural pesticide, such as wettable powder, emulsifiable concentrate, water-soluble powder, dust, granular formulation, suspension concentrate etc.

As solid carriers, cereal flours such as soy bean flour, wheat flour etc., ground minerals such as diatomaceous earth, apatite, gypsum, talc, bentonite clay etc. may be used.

As liquid carriers, vegetable oil, mineral oil, petroleum such as kerosine and solvent naphtha, xylene, cyclohexane, cyclohexanone, dimethylformamide, dimethylsulfoxide, trichloroethylene, methylisobutyl ketone and water may be used.

A surfactant may, if necessary, be added in order to give a homogeneous and stable formulation.

The concentration of the active ingredient in the composition may vary according to type of formulation, and is, for example, in the range of 5 ∿ 80 weight percent, preferably 10 ∿ 70 weight percent, in wettable powder; 5 ∿ 30 weight percent, preferably 10 ∿ 20 weight percent, in emulsifiable concentrate; 5 ∿ 80 weight percent, preferably 30 ∿ 60 weight percent, in water-soluble powder; 1 ∿ 10 weight percent, preferably 2 ∿ 5 weight percent in dust; 5 ∿ 40 weight percent, preferably 10 ∿ 30 weight percent in suspension concentrate; 1 ∿ 10 weight percent, preferably 2 ∿ 5 weight percent in granular formulation.

The wettable powder, the emulsifiable concentrate, water soluble-powder and suspension concentrate are diluted with water to the specified concentrations and used as an aqueous suspension or an aqueous emulsion to spray over the plants.

The dust and granular formulation are directly used for dusting or applying the plants.

Examples of the composition of this invention are as mentioned below, but the scope of the invention shall not be limitted to those:

Example 9 Emulsifiable Concentrate:

| | |
|---|---|
| Compound of this invention | 30 parts by weight |
| Dimethylformamide | 30 " |
| Xylene | 33 " |
| Polyoxyethylene alkylaryl ether | 7 " |

Those are mixed together to provide an emulsifiable concentrate containing 30% of active ingredient. At use, it is diluted with water to obtain a desired concentration of the compound of this invention in the emulsion.

Example 10 Wettable Powder:

| | |
|---|---|
| Compound of this invention | 40 perts by weight |
| Diatomaceous earth | 53 " |
| Higher alcohol sulfate ester | 4 " |
| Alkylnaphthalene sulfonic acid ester | 3 " |

Those are mixed together to provide a wettable powder containing 40% of active ingredient. At use, it is diluted with water to obtain a desired concentration of the compound of this invention in the suspension.

Example 11 Wettable Powder:

| | | |
|---|---|---|
| Compound of this invention | 50 | parts by weight |
| Benzimidazole-thiophanate series compound | 12.5 | " |
| White carbon | 32.5 | " |
| Higher fatty acid sodium salt | 3 | " |
| Calcium lignin sulfonate | 2 | " |

Those are mixed to provide a wettable powder containing 62.5% of active ingredient. At use, it is diluted with water to obtain a suspension at the desired concentration of the compounds concerned.

Example 12 Wettable Powder:

| | | |
|---|---|---|
| Compound of this invention | 25 | parts by weight |
| Benzimidazole-thiophanate series compound | 25 | " |
| White carbon | 45 | " |
| Higher fatty acid sodium salt | 3 | " |
| Calcium lignin sulfonate | 2 | " |

Those are mixed to provide a wettable powder containing 50% of active ingredient. At use, it is diluted with water to obtain a suspension at the desired concentration of the compounds concerned.

Example 13 Wettable powder:

| | | |
|---|---|---|
| Compound of this invention | 50 | parts by weight |
| Benzimidazole-thiophanate series compound | 10 | " |
| White carbon | 35 | " |

| | |
|---|---|
| Higher fatty acid sodium salt | 3 parts by weight |
| Calcium lignin sulfonate | 2 " |

Those are mixed to provide a wettable powder containing 60% of active ingredient. At use, it is diluted with water to obtain a suspension at the desired concentration of the compounds concerned.

Example 14 Dust:

| | |
|---|---|
| Compound of this invention | 10 parts by weight |
| Talc | 89 " |
| Polyoxyethylene alkylaryl ether | 1 " |

Those are mixed together to provide a dust containing 10% of active ingredient.

Further, it goes without saying that the compound of this invention or Mixed Chemicals indicate a sufficient fungicidal, insecticidal or acaricidal efficacy, but, in those composition, one kind or two kinds or more of other fungicidal, insecticidal or acaricidal compounds may be mixed (hereinafter called as "mixed composition"), because the compound of this invention or Mixed Chemicals might indicate an insufficient or inferior effect to some kinds of fungi, insects or acari.

Typical examples of fungicidal, insecticidal or acaricidal compounds usable together with the compound of this invention or Mixed Chemical in the mixed composition are set forth below:

[Fungicide]

Captan, TMTD, zineb, manneb, manzeb, TPN, phenfram, furabax, Alliette, prothiocarb, triadimeton, triadimenol, polyoxine, tridemorph, metalaxyl, furalaxyl, triforine, isoprothiolane, probenazole, blasticidin-S, kasugamycin, validamycin A, PCNB, iprodione, vinclozolin, procimidone, basic cupper chloride, basic cupper sulfate, triphenyltin hydroxide, quinomethionate, propamocarb, binapacryl.

[Insecticide and Acaricide]

BCPE, chlorobenzylate, chloropropylate, prochlonol, phenylsobromolate, dicofol, chlorophenamidine, amitraz, BPPS, PPPS, benzomate, cyhexatin, polynactin, thioquinox, CPCBS, tetradifon, tetrasul, cycloplate, phenproxide, Kayahope, lime-polysulfide, 3-n-dodecyl-1,4-naphthoquinone-2-yl acetate, fenthion, fenitrothion, diazinon, chloropyrifos, ESP, vamidothion, phentkoate, dimethoate, formothion, malathion, DEP, thiometon, phosmet, menazon, dichlorvos, acephate, EPBP, dialifor, methylparathion, oxydemethon-methyl, ethion, aldicarb, propoxur, permethrin, cypermethrin, decamethrin, phenvalerate, phenpropathrin, pyrethrin, allethrin, tetramethrin, resmethrin, dimethrin, proparthrin, prothrin, 3-phenoxybenzyl-2,2-dichloro-1-(4-ethoxyphenyl)-1-cyclopropane carboxylate, α-cyano-3-phenoxybenzyl-(RS)-2-(4-trichloromethoxyphenyl)-3-methyl butylate, (RS)-α-cyano-3-phenoxybenzyl-(RS)-2-(2-chloro-4-trichloromethylanilino)-3-methyl butylate, petroleum oil.

The fungicidal, insecticidal and acaricidal activity of the compound or Mixed Chemicals are illustrated by the following tests.

Test 1 Gray mould (Botrytis cinerea) of kidney bean:

The seedling of kidney bean (Variety: Nagauzura) was cultivated about 3 weeks, and the main leaves cut therefrom were immersed for about 30 seconds in a aqueous solution having a specified concentration of active ingredient(s) which was prepared from the wettable powder formulated in accordance with (mutatis mutandis) the method of Example 10 or Example 13, and then air-dried.

The dried leaves were inoculated with mycelium of Gray mould of kidney bean (Botrytis cinerea) Chemical Resistant Fungi, Chemical Sensitive Fungi, Mixed Fungi or Combined Fungi and kept in the humid room at 20°C.

The disease occurrence degree for each leaf treated with said aqueous solution was investigated at the 4th day after the inoculation.

In accordance with the investigating standard set forth below, the disease indexes were measured and preventive value for each compound or each of Mixed Chemicals was calculated by the calculation formula of preventive value (%).

| | | | | | | |
|---|---|---|---|---|---|---|
| Diameter of disease lesion (mm) | 0 | 1∿4 | 5∿10 | 11∿18 | 19∿29 | 30≧ |
| Disease index | 0 | 0.5 | 1 | 2 | 3 | 4 |

$$\text{Preventive Value (\%)} = \left(1 - \frac{\text{Average disease index in treated zone}}{\text{Average disease index in untreated sone}}\right) \times 100$$

The results are shown in Table 2 (2-1 to 2-3) as follows:

Table 2

2-1 Activity of the compound

| Compound No. | Concentration of Active ingredient (ppm) | Preventive Value (%) | |
|---|---|---|---|
| | | Chemical Resistant Fungi | Chemical Sensitive Fungi |
| 5 | 200 | 100 | 0 |
| 8 | " | " | " |
| 9 | " | " | 8 |
| 10 | " | " | 0 |
| 13 | " | " | 8 |
| 14 | " | " | " |
| 15 | " | 87 | 0 |
| 18 | " | 97 | " |
| 24 | " | 96 | " |
| 31 | " | 100 | " |
| 32 | " | " | " |
| 33 | " | 98 | " |
| 34 | " | 100 | " |
| 35 | " | " | " |
| 36 | " | 91 | " |
| 37 | " | 99 | " |
| 44 | " | 84 | " |
| 45 | " | 100 | " |
| 48 | " | 88 | " |
| 49 | " | 92 | " |
| 50 | " | 81 | " |
| 54 | " | 100 | " |

(to be cont'd)



| | | | |
|---|---|---|---|
| 56 | 200 | 98 | 0 |
| 60 | " | 100 | " |
| 61 | " | 98 | " |
| 66 | " | 100 | " |
| 77 | " | " | 25 |
| 79 | " | 96 | 0 |
| 80 | " | 100 | " |
| 81 | " | " | 17 |
| 83 | " | " | 25 |
| 84 | " | 96 | 0 |
| 85 | " | 100 | " |
| 86 | " | " | 25 |
| 87 | " | " | 0 |
| 88 | " | " | 8 |
| 89 | " | " | 0 |
| 90 | " | " | " |
| 92 | " | " | 8 |
| 93 | " | " | 0 |
| 94 | " | " | " |
| 95 | " | " | " |
| 96 | " | " | " |
| 97 | " | 90 | " |
| 99 | " | 99 | " |
| 104 | " | 100 | 25 |
| 108 | " | " | 0 |
| 109 | " | " | " |
| 110 | " | " | " |
| 111 | " | " | " |

| | | | |
|---|---|---|---|
| 112 | 200 | 100 | 0 |
| 113 | " | " | " |
| 114 | " | " | " |
| 115 | " | " | " |
| 116 | " | " | " |
| 117 | " | " | " |
| 118 | " | " | " |
| 119 | " | " | " |
| 120 | " | " | " |
| 121 | " | " | " |
| 122 | " | " | " |
| 123 | " | " | " |
| 124 | " | " | " |
| 125 | " | " | " |
| 126 | " | " | " |
| 127 | " | " | " |
| 128 | " | " | " |
| 129 | " | " | " |
| 131 | " | 98 | " |
| 133 | " | 100 | " |
| 134 | " | " | " |
| 135 | " | " | " |
| 136 | " | " | " |
| 139 | " | " | " |
| 140 | " | " | 8 |
| 141 | " | " | 0 |
| 142 | " | " | " |
| 146 | " | " | " |

(to be cont'd)



| | | | |
|---|---|---|---|
| 147 | 200 | 100 | 20 |
| 148 | " | " | 25 |
| 149 | " | " | 0 |
| 150 | " | " | " |
| 151 | " | 98 | " |
| 154 | " | 100 | " |
| 160 | " | " | " |
| 161 | " | " | " |
| 175 | " | 89 | " |
| 178 | " | 100 | " |
| 199 | " | 95 | 8 |
| 201 | " | 100 | 0 |
| 202 | " | " | " |
| 208 | " | " | 8 |
| 209 | " | " | 0 |
| 230 | " | " | 0 |
| 231 | " | " | 17 |
| 238 | " | 94 | 8 |
| 239 | " | 97 | 0 |
| 241 | " | 100 | " |
| 242 | " | " | " |
| 246 | " | " | " |
| 247 | " | " | " |
| 248 | " | " | " |
| 249 | " | " | " |
| 250 | " | " | " |
| 251 | " | " | " |
| 253 | " | " | " |

(to be cont'd)



| | | | |
|---|---|---|---|
| 254 | 200 | 100 | 0 |
| 255 | " | " | " |
| 256 | " | " | " |
| 257 | " | " | " |
| 258 | " | " | " |
| 261 | " | " | 0 |
| 264 | " | " | " |
| 267 | " | " | " |
| 268 | " | " | 8 |
| 269 | " | " | 0 |
| 272 | " | 84 | " |
| 275 | " | 92 | " |
| 282 | " | 100 | " |
| 283 | " | " | " |
| 284 | " | " | " |
| 285 | " | " | " |
| 286 | " | " | " |
| 287 | " | 87 | " |
| 288 | " | 100 | 25 |
| 290 | " | " | 0 |
| 291 | " | " | " |
| 292 | " | " | 25 |
| 293 | " | 96 | " |
| 294 | " | 99 | 0 |
| 295 | " | 100 | 8 |
| 296 | " | " | 0 |
| 298 | " | " | " |
| 304 | " | " | " |

| | | | |
|---|---|---|---|
| 305 | 200 | 100 | 0 |
| 309 | " | " | 25 |
| 317 | " | " | 100 |
| 320 | " | " | " |
| 321 | " | " | " |
| 322 | " | 91 | 81 |
| 325 | " | 100 | 100 |
| 326 | " | " | " |
| 327 | " | – | 84 |
| 337 | " | 94 | 90 |
| 338 | " | – | 81 |
| 344 | " | 100 | 100 |
| 345 | " | – | 88 |
| 348 | " | 87 | 87 |
| 398 | " | 99 | 0 |
| 399 | " | 100 | 25 |
| 400 | " | " | 0 |
| 401 | " | " | " |
| 402 | " | " | " |
| 403 | " | " | " |
| 404 | " | " | " |
| 405 | " | " | " |
| 406 | " | " | 25 |
| 408 | " | 99 | 0 |
| 410 | " | 100 | " |
| 411 | " | " | " |
| 413 | " | 95 | " |
| 414 | " | 100 | " |

(to be cont'd)



| | | | |
|---|---|---|---|
| 415 | 200 | 100 | 0 |
| 417 | " | " | " |
| 418 | " | " | " |
| 419 | " | " | " |
| 420 | " | " | 8 |
| 421 | " | " | 0 |
| 422 | " | " | " |
| 424 | " | " | " |
| 425 | " | " | " |
| 427 | " | " | " |
| 428 | " | " | " |
| 430 | " | " | " |
| 431 | " | " | 17 |
| 432 | " | " | 8 |
| 433 | " | " | 0 |
| 434 | " | " | " |
| 435 | " | " | " |
| 436 | " | " | " |
| 437 | " | " | " |
| 438 | " | 98 | 25 |
| 439 | " | 100 | 0 |
| 442 | " | " | " |
| 443 | " | " | " |
| 444 | " | " | " |
| 445 | " | " | " |
| 446 | " | " | " |
| 447 | " | " | 100 |
| 448 | " | - | 85 |

| | | | |
|---|---|---|---|
| 450 | 200 | 91 | 87 |
| 454 | " | 100 | 0 |
| Comparative Compound *1 | " | 0 | 100 |
| " 2 | " | " | " |
| " 3 | " | 82 | 82 |
| " 4 | " | 25 | 12 |

* Comparative Compound 1: thiophanate methyl

" 2: benomyl

" 3: dichlofluanid

" 4:

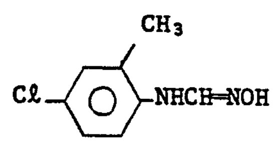

(U.S.P. 4,237,168)

Table 2

2-2 Activity of Mixed Chemicals

| Compound No. | Concentration of Active ingredient (ppm) | Preventive Value (%) | | |
|---|---|---|---|---|
| | | Chemical Resistant Fungi | Mixed Fungi | Chemical Sensitive Fugi |
| 6<br>BT-1* | 50<br>10 | 100 | 100 | 100 |
| 6<br>BT-2** | " | " | " | " |
| 6<br>BT-3*** | " | " | " | " |
| 7<br>BT-1 | " | " | " | " |
| 7<br>BT-2 | " | " | " | " |
| 7<br>BT-3 | " | " | " | " |
| 19<br>BT-1 | " | " | " | " |
| 19<br>BT-2 | " | " | " | " |
| 19<br>BT-3 | " | " | " | " |
| 23<br>BT-1 | " | " | " | " |
| 23<br>BT-2 | " | " | " | " |
| 23<br>BT-3 | " | " | " | " |

(to be cont'd)



| | | | | |
|---|---|---|---|---|
| 29<br>BT-1 | 50<br>10 | 100 | 100 | 100 |
| 29<br>BT-2 | " | " | " | " |
| 29<br>BT-3 | " | " | " | " |
| 30<br>BT-1 | " | " | " | " |
| 30<br>BT-2 | " | " | " | " |
| 30<br>BT-3 | " | " | " | " |
| 46<br>BT-1 | " | " | " | " |
| 46<br>BT-2 | " | " | " | " |
| 46<br>BT-3 | " | " | " | " |
| 47<br>BT-1 | " | " | " | " |
| 47<br>BT-2 | " | " | " | " |
| 47<br>BT-3 | " | " | " | " |
| 62<br>BT-1 | " | " | " | " |
| 62<br>BT-2 | " | " | " | " |
| 62<br>BT-3 | " | " | " | " |
| 78<br>BT-1 | " | " | " | " |

(to be cont'd)



| | | | | |
|---|---|---|---|---|
| 78<br>BT-2 | 50<br>10 | 100 | 100 | 100 |
| 78<br>BT-3 | " | " | " | " |
| 91<br>BT-1 | " | " | " | " |
| 91<br>BT-2 | " | " | " | " |
| 91<br>BT-3 | " | " | " | " |
| 98<br>BT-1 | " | " | " | " |
| 98<br>BT-2 | " | " | " | " |
| 98<br>BT-3 | " | " | " | " |
| 101<br>BT-1 | " | " | " | " |
| 101<br>BT-2 | " | " | " | " |
| 101<br>BT-3 | " | " | " | " |
| 103<br>BT-1 | " | " | " | " |
| 103<br>BT-2 | " | " | " | " |
| 103<br>BT-3 | " | " | " | " |
| 105<br>BT-1 | " | " | " | " |
| 105<br>BT-2 | " | " | " | " |

(to be cont'd)



| | | | | |
|---|---|---|---|---|
| 105<br>BT-3 | 50<br>10 | 100 | 100 | 100 |
| 106<br>BT-1 | " | " | " | " |
| 106<br>BT-2 | " | " | " | " |
| 106<br>BT-3 | " | " | " | " |
| 107<br>BT-1 | " | " | " | " |
| 107<br>BT-2 | " | " | " | " |
| 107<br>BT-3 | " | " | " | " |
| 132<br>BT-1 | " | " | " | " |
| 132<br>BT-2 | " | " | " | " |
| 132<br>BT-3 | " | " | " | " |
| 143<br>BT-1 | " | " | " | " |
| 143<br>BT-2 | " | " | " | " |
| 143<br>BT-3 | " | " | " | " |
| 145<br>BT-1 | " | " | " | " |
| 145<br>BT-2 | " | " | " | " |
| 145<br>BT-3 | " | " | " | " |

| | | | | |
|---|---|---|---|---|
| 153<br>BT-1 | 50<br>10 | 100 | 100 | 100 |
| 153<br>BT-2 | " | " | " | " |
| 153<br>BT-3 | " | " | " | " |
| 162<br>BT-1 | " | " | " | " |
| 162<br>BT-2 | " | " | " | " |
| 162<br>BT-3 | " | " | " | " |
| 165<br>BT-1 | " | " | " | " |
| 165<br>BT-2 | " | " | " | " |
| 165<br>BT-3 | " | " | " | " |
| 200<br>BT-1 | " | " | " | " |
| 200<br>BT-2 | " | " | " | " |
| 200<br>BT-3 | " | " | " | " |
| 229<br>BT-1 | " | " | " | " |
| 229<br>BT-2 | " | " | " | " |
| 229<br>BT-3 | " | " | " | " |
| 236<br>BT-1 | " | " | " | " |

(to be cont'd)



| | | | | |
|---|---|---|---|---|
| 236<br>BT-2 | 50<br>10 | 100 | 100 | 100 |
| 236<br>BT-3 | " | " | " | " |
| 243<br>BT-1 | " | " | " | " |
| 243<br>BT-2 | " | " | " | " |
| 243<br>BT-3 | " | " | " | " |
| 244<br>BT-1 | " | " | " | " |
| 244<br>BT-2 | " | " | " | " |
| 244<br>BT-3 | " | " | " | " |
| 245<br>BT-1 | " | " | " | " |
| 245<br>BT-2 | " | " | " | " |
| 245<br>BT-3 | " | " | " | " |
| 252<br>BT-1 | " | " | " | " |
| 252<br>BT-2 | " | " | " | " |
| 252<br>BT-3 | " | " | " | " |
| 259<br>BT-1 | " | " | " | " |
| 259<br>BT-2 | " | " | " | " |

(to be cont'd)



| | | | | |
|---|---|---|---|---|
| 259<br>BT-3 | 50<br>10 | 100 | 100 | 100 |
| 260<br>BT-1 | " | " | " | " |
| 260<br>BT-2 | " | " | " | " |
| 260<br>BT-3 | " | " | " | " |
| 262<br>BT-1 | " | " | " | " |
| 262<br>BT-2 | " | " | " | " |
| 262<br>BT-3 | " | " | " | " |
| 263<br>BT-1 | " | " | " | " |
| 263<br>BT-2 | " | " | " | " |
| 263<br>BT-3 | " | " | " | " |
| 265<br>BT-1 | " | " | " | " |
| 265<br>BT-2 | " | " | " | " |
| 265<br>BT-3 | " | " | " | " |
| 270<br>BT-1 | " | " | " | " |
| 270<br>BT-2 | " | " | " | " |
| 270<br>BT-3 | " | " | " | " |

(to be cont'd)



| | | | | |
|---|---|---|---|---|
| 281<br>BT-1 | 50<br>10 | 100 | 100 | 100 |
| 281<br>BT-2 | " | " | " | " |
| 281<br>BT-3 | " | " | " | " |
| 289<br>BT-1 | " | " | " | " |
| 289<br>BT-2 | " | " | " | " |
| 289<br>BT-3 | " | " | " | " |
| 297<br>BT-1 | " | " | " | " |
| 297<br>BT-2 | " | " | " | " |
| 297<br>BT-3 | " | " | " | " |
| 310<br>BT-1 | " | " | " | " |
| 310<br>BT-2 | " | " | " | " |
| 310<br>BT-3 | " | " | " | " |
| 407<br>BT-1 | " | " | " | " |
| 407<br>BT-2 | " | " | " | " |
| 407<br>BT-3 | " | " | " | " |
| 409<br>BT-1 | " | " | " | " |

(to be cont'd)



| | | | | |
|---|---|---|---|---|
| 409<br>BT-2 | 50<br>10 | 100 | 100 | 100 |
| 409<br>BT-3 | " | " | " | " |
| 416<br>BT-1 | " | " | " | " |
| 416<br>BT-2 | " | " | " | " |
| 416<br>BT-3 | " | " | " | " |
| 417<br>BT-1 | " | " | " | " |
| 417<br>BT-2 | " | " | " | " |
| 417<br>BT-3 | " | " | " | " |
| 423<br>BT-1 | " | " | " | " |
| 423<br>BT-2 | " | " | " | " |
| 423<br>BT-3 | " | " | " | " |
| 425<br>BT-1 | " | " | " | " |
| 425<br>BT-2 | " | " | " | " |
| 425<br>BT-3 | " | " | " | " |
| 440<br>BT-1 | " | " | " | " |
| 440<br>BT-2 | " | " | " | " |

| | | | | |
|---|---|---|---|---|
| 440<br>BT-3 | 50<br>10 | 100 | 100 | 100 |
| 441<br>BT-1 | " | " | " | " |
| 441<br>BT-2 | " | " | " | " |
| 441<br>BT-3 | " | " | " | " |
| 6 | 200<br>50 | 100<br>35 | 10<br>0 | 0<br>0 |
| 7 | " | 100<br>40 | 20<br>0 | " |
| 19 | " | 100<br>28 | 10<br>0 | " |
| 23 | " | 100<br>55 | " | " |
| 29 | " | 100<br>35 | " | " |
| 30 | " | 100<br>55 | 7<br>0 | " |
| 46 | " | 100<br>35 | 20<br>0 | " |
| 47 | " | 98<br>20 | 10<br>0 | 7<br>0 |
| 62 | " | 100<br>55 | " | 0<br>0 |
| 78 | " | 90<br>20 | 0<br>0 | " |
| 91 | " | 100<br>35 | 15<br>0 | 10<br>0 |
| 98 | " | 100<br>55 | 10<br>0 | 0<br>0 |

| | | | | |
|---|---|---|---|---|
| 101 | 200<br>50 | 100<br>50 | 0<br>0 | 0<br>0 |
| 103 | " | 100<br>55 | " | " |
| 105 | " | 100<br>40 | 10<br>0 | " |
| 106 | " | 100<br>20 | 7<br>0 | " |
| 107 | " | 100<br>50 | 15<br>0 | " |
| 132 | " | 100<br>20 | 7<br>0 | " |
| 143 | " | 100<br>50 | 30<br>10 | " |
| 145 | " | 100<br>45 | 15<br>0 | 8<br>0 |
| 153 | " | 100<br>35 | 10<br>0 | 0<br>0 |
| 162 | " | 100<br>25 | 15<br>0 | " |
| 165 | " | 100<br>30 | 10<br>0 | " |
| 200 | " | 100<br>45 | 15<br>0 | 8<br>0 |
| 229 | " | 100<br>40 | 10<br>0 | 0<br>0 |
| 236 | " | 100<br>50 | " | " |
| 243 | " | " | 20<br>0 | " |
| 244 | " | " | 10<br>0 | " |

(to be cont'd)



| | | | | |
|---|---|---|---|---|
| 245 | 200<br>50 | 100<br>30 | 10<br>0 | 0<br>0 |
| 252 | " | 100<br>10 | 7<br>0 | " |
| 259 | " | 100<br>40 | 15<br>0 | " |
| 260 | " | 100<br>50 | 10<br>0 | " |
| 262 | " | 100<br>55 | " | " |
| 263 | " | " | 0<br>0 | " |
| 265 | " | 100<br>45 | 15<br>0 | " |
| 270 | " | 100<br>50 | " | " |
| 281 | " | 100<br>10 | 7<br>0 | " |
| 289 | " | 100<br>20 | 0<br>0 | " |
| 297 | " | 100<br>15 | 7<br>0 | " |
| 310 | " | 100<br>20 | " | " |
| 407 | " | 100<br>30 | 15<br>0 | " |
| 409 | " | " | 5<br>0 | " |
| 416 | " | 100<br>50 | 10<br>0 | " |
| 417 | " | 100<br>55 | 20<br>0 | " |

| | | | | |
|---|---|---|---|---|
| 423 | 200<br>50 | 99<br>40 | 0<br>0 | 0<br>0 |
| 425 | " | 100<br>55 | " | " |
| 440 | " | 100<br>30 | 20<br>0 | " |
| 441 | " | 100<br>40 | " | " |
| BT-1 | 50<br>10 | 0<br>0 | " | 100<br>30 |
| BT-2 | " | " | 30<br>0 | 100<br>40 |
| BT-3 | " | " | 40<br>0 | " |

* BT-1 :

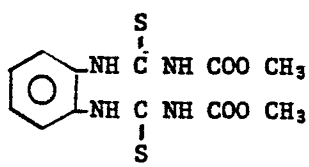

(thiophanate methyl)

** BT-2 :

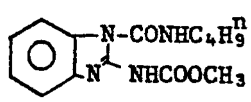

(benomyl)

*** BT-3 :

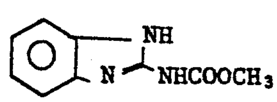

(carbendazim)

Table 2

2-3 Activity of the compound against Combined Fungi

| Compound No. | Concentration of Active ingredient (ppm) | Preventive Value (%) | | | |
|---|---|---|---|---|---|
| | | BT/S & CI/S | BT/S & CI/R | BT/R & CI/S | BT/R & CI/R |
| 7 | 200 | 0 | 0 | 100 | 100 |
| 29 | " | " | " | " | " |
| 30 | " | " | " | " | " |
| 81 | " | " | " | " | " |
| 139 | " | " | " | " | " |
| 242 | " | " | " | " | " |
| 243 | " | " | " | " | " |
| 409 | " | " | " | " | " |
| 414 | " | " | " | " | " |
| Comparative Compound 1* | " | 100 | 100 | 0 | 0 |
| " 2 | " | " | " | " | " |
| " 3 | " | " | 0 | 100 | " |
| " 4 | " | " | " | " | " |

* Comparative Compound 1 : thiophanate methyl
" 2 : carbendazim
" 3 : vinclozolin
" 4 : iprodione

Test 2 Powdery mildew (Sphaerotheca fuliginea) of cucumber:

A sufficient amount of the chemical solution having a specified concentration prepared from a wettable powder of each test compound was sprayed over the seedling of cucumber (Variety: Satsukimidori) cultivated in a pot for about 3 weeks in a green house.

The seedling was air-dried and then inoculated with conidia of powdery mildew (Spnaerotheca fuliginer) Chemical Resistant Fungi, Chemical Sensitive Fungi or Mixed Fungi.

Each seedling pot was separately or distantly kept in the greenhouse at about 25°C.

The disease occurrence status was investigated at 10th day after the inoculation.

The disease index was calculated with the following method. In the other words, disease index was classified as 0, 1, 2, 3 ... 10 in compliance with appearance of disease spots on each leaf.

The preventive value of each compound or each of Mixed Chemicals were calculated by the following calculating formula of the preventive value.

| Disease index | Occurrence status of disease lesion or spots |
|---|---|
| 0 ... | Any disease lesion or any disease spot is not entirely indentified on leaf surface. |
| 1 ... | One to five pieces of disease spots appeare on leaf surface. |

2 ... 6 to 10 pieces of disease spots appeare on leaf surface.

3 ... 30% of surface area of the leaf is affected with the disease lesion or spots.

4 ... 31 - 40% of surface area of leaf is affected with the lesion or spots.

5 ... 41 - 50% of surface area of leaf is affected with the lesion or spots.

6 ... 51 - 60% of surface area of leaf is affected with the lesion or spots.

7 ... 61 - 70% of surface area of leaf is affected with the lesion or spots.

8 ... 71 - 80% of surface area of leaf is affected with the lesion or spots.

9 ... 81 - 90% of surface area of leaf is affected with the lesion or spots.

10 ... 91 - 100% of surface area of leaf is affected with the lesion or spots.

$$\text{Preventive value (\%)} = \left(1 - \frac{\text{Average disease index in treated zone}}{\text{Average disease index in untreated zone}}\right) \times 100$$

The results are shown in Table 3 (3-1 and 3-2) as follows:

Table 3

3-1

Activity of the compound

| Compound No. | Concentration of Active ingredient (ppm) | Preventive Value (%) | |
|---|---|---|---|
| | | Chemical Resistant Fungi | Chemical Sensitive Fungi |
| 6 | 200 | 100 | 0 |
| 8 | " | " | " |
| 9 | " | " | " |
| 10 | " | " | " |
| 13 | " | 95 | " |
| 14 | " | 100 | " |
| 16 | " | " | " |
| 18 | " | " | " |
| 29 | " | " | " |
| 31 | " | " | " |
| 32 | " | " | " |
| 33 | " | " | " |
| 34 | " | " | " |
| 35 | " | " | " |
| 36 | " | " | " |
| 37 | " | " | " |
| 38 | " | " | " |
| 39 | " | " | " |
| 42 | " | 85 | " |
| 48 | " | 100 | " |
| 49 | " | 95 | " |
| 50 | " | " | " |

| | | | |
|---|---|---|---|
| 51 | 200 | 100 | 0 |
| 52 | " | 95 | " |
| 53 | " | 100 | " |
| 54 | " | " | " |
| 56 | " | " | " |
| 60 | " | " | " |
| 61 | " | 85 | " |
| 66 | " | 100 | " |
| 78 | " | " | " |
| 79 | " | 95 | " |
| 80 | " | 100 | " |
| 81 | " | " | " |
| 82 | " | 90 | " |
| 83 | " | " | " |
| 84 | " | 95 | " |
| 85 | " | 100 | " |
| 88 | " | " | " |
| 89 | " | " | " |
| 90 | " | 90 | " |
| 92 | " | 95 | " |
| 93 | " | 100 | " |
| 94 | " | " | " |
| 95 | " | " | " |
| 96 | " | " | " |
| 97 | " | " | " |
| 98 | " | " | " |
| 99 | " | 85 | " |
| 100 | " | 95 | " |

(to be cont'd)





| | | | |
|---|---|---|---|
| 102 | 200 | 95 | 0 |
| 104 | " | 100 | " |
| 106 | " | 95 | " |
| 109 | " | " | " |
| 110 | " | 100 | " |
| 111 | " | " | " |
| 112 | " | " | " |
| 113 | " | 95 | " |
| 114 | " | 100 | " |
| 115 | " | " | " |
| 116 | " | " | " |
| 117 | " | " | " |
| 118 | " | " | " |
| 119 | " | " | " |
| 120 | " | " | " |
| 121 | " | " | " |
| 122 | " | " | " |
| 123 | " | " | " |
| 124 | " | " | " |
| 125 | " | " | " |
| 126 | " | " | " |
| 127 | " | " | " |
| 128 | " | 90 | " |
| 129 | " | 100 | " |
| 130 | " | " | " |
| 132 | " | 90 | " |
| 133 | " | 95 | " |
| 134 | " | 100 | " |

| | | | |
|---|---|---|---|
| 135 | 200 | 100 | 0 |
| 139 | " | " | " |
| 140 | " | " | " |
| 141 | " | " | " |
| 142 | " | 95 | " |
| 147 | " | 100 | " |
| 148 | " | " | " |
| 149 | " | 95 | " |
| 160 | " | 100 | " |
| 166 | " | 90 | " |
| 168 | " | 95 | " |
| 182 | " | " | " |
| 200 | " | 90 | " |
| 229 | " | " | " |
| 233 | " | 100 | " |
| 234 | " | 95 | " |
| 241 | " | 100 | " |
| 249 | " | 95 | " |
| 250 | " | 100 | " |
| 251 | " | " | " |
| 253 | " | " | " |
| 254 | " | " | " |
| 255 | " | " | " |
| 256 | " | " | " |
| 260 | " | " | " |
| 268 | " | 95 | " |
| 285 | " | 100 | " |
| 286 | " | " | " |

(to be cont'd)



| | | | |
|---|---|---|---|
| 287 | 200 | 100 | 0 |
| 295 | " | 90 | " |
| 296 | " | " | " |
| 298 | " | 100 | " |
| 304 | " | " | " |
| 305 | " | " | " |
| 309 | " | 95 | " |
| 310 | " | 100 | " |
| 400 | " | 95 | " |
| 404 | " | 90 | " |
| 406 | " | 95 | " |
| 407 | " | 100 | " |
| 408 | " | " | " |
| 409 | " | " | " |
| 410 | " | 85 | " |
| 411 | " | 95 | " |
| 414 | " | 90 | " |
| 415 | " | 95 | " |
| 416 | " | 90 | " |
| 417 | " | 95 | " |
| 418 | " | 100 | " |
| 419 | " | " | " |
| 420 | " | " | " |
| 421 | " | " | " |
| 422 | " | " | " |
| 423 | " | " | " |
| 424 | " | " | " |
| 425 | " | " | " |

(to be cont'd)

| | | | |
|---|---|---|---|
| 427 | 200 | 100 | 0 |
| 428 | " | 90 | " |
| 429 | " | 100 | " |
| 430 | " | " | " |
| 432 | " | 95 | " |
| 434 | " | 100 | " |
| 435 | " | " | " |
| 440 | " | " | " |
| 441 | " | " | " |
| 442 | " | " | " |
| 443 | " | " | " |
| 445 | " | " | " |
| 454 | " | " | " |
| Comparative Compound 1* | " | 0 | 100 |
| " 2 | " | " | " |
| " 3 | " | 10 | 5 |

* Comparative Compound 1 : thiophanate methyl

" 2 : carbendazim

" 3 : (U.S.P. 4,237,168)

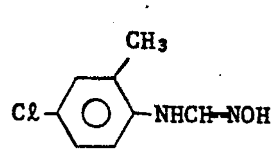

Table 3

3-2

Activity of Mixed Chemicals

| Compound No. | Concentration of Active ingredient (ppm) | Preventive Value (%) | | |
|---|---|---|---|---|
| | | Chemical Resistant Fungi | Mixed Fungi | Chemical Sensitive Fugi |
| 7<br>BT-1 | 50<br>10 | 100 | 100 | 100 |
| 7<br>BT-4 | " | " | " | " |
| 7<br>BT-5 | " | " | " | " |
| 7<br>BT-6 | " | " | " | " |
| 19<br>BT-1 | " | " | " | " |
| 19<br>BT-4 | " | " | " | " |
| 19<br>BT-5 | " | " | " | " |
| 19<br>BT-6 | " | " | " | " |
| 23<br>BT-1 | " | " | " | " |
| 23<br>BT-4 | " | " | " | " |
| 23<br>BT-5 | " | " | " | " |
| 23<br>BT-6 | " | " | " | " |
| 30<br>BT-1 | " | " | " | " |

(to be cont'd)



| | | | | |
|---|---|---|---|---|
| 30<br>BT-4 | 50<br>10 | 100 | 100 | 100 |
| 30<br>BT-5 | " | " | " | " |
| 30<br>BT-6 | " | " | " | " |
| 47<br>BT-1 | " | " | " | " |
| 47<br>BT-4 | " | " | " | " |
| 47<br>BT-5 | " | " | " | " |
| 47<br>BT-6 | " | " | " | " |
| 62<br>BT-1 | " | " | " | " |
| 62<br>BT-4 | " | " | " | " |
| 62<br>BT-5 | " | " | " | " |
| 62<br>BT-6 | " | " | " | " |
| 91<br>BT-1 | " | " | " | " |
| 91<br>BT-4 | " | " | " | " |
| 91<br>BT-5 | " | " | " | " |
| 91<br>BT-6 | " | " | " | " |
| 101<br>BT-1 | " | " | " | " |

(to be cont'd)



| | | | | |
|---|---|---|---|---|
| 101<br>BT-4 | 50<br>10 | 100 | 100 | 100 |
| 101<br>BT-5 | " | " | " | " |
| 101<br>BT-6 | " | " | " | " |
| 105<br>BT-1 | " | " | " | " |
| 105<br>BT-4 | " | " | " | " |
| 105<br>BT-5 | " | " | " | " |
| 105<br>BT-6 | " | " | " | " |
| 107<br>BT-1 | " | " | " | " |
| 107<br>BT-4 | " | " | " | " |
| 107<br>BT-5 | " | " | " | " |
| 107<br>BT-6 | " | " | " | " |
| 143<br>BT-1 | " | " | " | " |
| 143<br>BT-4 | " | " | " | " |
| 143<br>BT-5 | " | " | " | " |
| 143<br>BT-6 | " | " | " | " |
| 165<br>BT-1 | " | " | " | " |

(to be cont'd)



| | | | | |
|---|---|---|---|---|
| 165<br>BT-4 | 50<br>10 | 100 | 100 | 100 |
| 165<br>BT-5 | " | " | " | " |
| 165<br>BT-6 | " | " | " | " |
| 243<br>BT-1 | " | " | " | " |
| 243<br>BT-4 | " | " | " | " |
| 243<br>BT-5 | " | " | " | " |
| 243<br>BT-6 | " | " | " | " |
| 244<br>BT-1 | " | " | " | " |
| 244<br>BT-4 | " | " | " | " |
| 244<br>BT-5 | " | " | " | " |
| 244<br>BT-6 | " | " | " | " |
| 245<br>BT-1 | " | " | " | " |
| 245<br>BT-4 | " | " | " | " |
| 245<br>BT-5 | " | " | " | " |
| 245<br>BT-6 | " | " | " | " |
| 252<br>BT-1 | " | " | " | " |

(to be cont'd)



| | | | | |
|---|---|---|---|---|
| 252<br>BT-4 | 50<br>10 | 100 | 100 | 100 |
| 252<br>BT-5 | " | " | " | " |
| 252<br>BT-6 | " | " | " | " |
| 262<br>BT-1 | " | " | " | " |
| 262<br>BT-4 | " | " | " | " |
| 262<br>BT-5 | " | " | " | " |
| 262<br>BT-6 | " | " | " | " |
| 263<br>BT-1 | " | " | " | " |
| 263<br>BT-4 | " | " | " | " |
| 263<br>BT-5 | " | " | " | " |
| 263<br>BT-6 | " | " | " | " |
| 267<br>BT-1 | " | " | " | " |
| 267<br>BT-4 | " | " | " | " |
| 267<br>BT-5 | " | " | " | " |
| 267<br>BT-6 | " | " | " | " |
| 269<br>BT-1 | " | " | " | " |

| | | | | |
|---|---|---|---|---|
| 269<br>BT-4 | 50<br>10 | 100 | 100 | 100 |
| 269<br>BT-5 | " | " | " | " |
| 269<br>BT-6 | " | " | " | " |
| 270<br>BT-1 | " | " | " | " |
| 270<br>BT-4 | " | " | " | " |
| 270<br>BT-5 | " | " | " | " |
| 270<br>BT-6 | " | " | " | " |
| 282<br>BT-1 | " | " | " | " |
| 282<br>BT-4 | " | " | " | " |
| 282<br>BT-5 | " | " | " | " |
| 282<br>BT-6 | " | " | " | " |
| 300<br>BT-1 | " | " | " | " |
| 300<br>BT-4 | " | " | " | " |
| 300<br>BT-5 | " | " | " | " |
| 300<br>BT-6 | " | " | " | " |
| 301<br>BT-1 | " | " | " | " |

(to be cont'd)



| | | | | |
|---|---|---|---|---|
| 301<br>BT-4 | 50<br>10 | 100 | 100 | 100 |
| 301<br>BT-5 | " | " | " | " |
| 301<br>BT-6 | " | " | " | " |
| 302<br>BT-1 | " | " | " | " |
| 302<br>BT-4 | " | " | " | " |
| 302<br>BT-5 | " | " | " | " |
| 302<br>BT-6 | " | " | " | " |
| 405<br>BT-1 | " | " | " | " |
| 405<br>BT-4 | " | " | " | " |
| 405<br>BT-5 | " | " | " | " |
| 405<br>BT-6 | " | " | " | " |
| 434<br>BT-1 | " | " | " | " |
| 434<br>BT-4 | " | " | " | " |
| 434<br>BT-5 | " | " | " | " |
| 434<br>BT-6 | " | " | " | " |
| 441<br>BT-1 | " | " | " | " |

| | | | | |
|---|---|---|---|---|
| 441<br>BT-4 | 50<br>10 | 100 | 100 | 100 |
| 441<br>BT-5 | " | " | " | " |
| 441<br>BT-6 | " | " | " | " |
| 7 | 200<br>50 | 100<br>40 | 10<br>0 | 0<br>0 |
| 19 | " | 100<br>30 | 15<br>0 | " |
| 23 | " | 100<br>40 | 25<br>0 | " |
| 30 | " | 100<br>45 | 30<br>0 | " |
| 47 | " | 100<br>40 | 25<br>0 | " |
| 62 | " | " | 15<br>0 | " |
| 91 | " | 100<br>45 | 25<br>0 | " |
| 101 | " | 100<br>20 | 10<br>0 | " |
| 105 | " | " | " | " |
| 107 | " | 100<br>25 | 15<br>0 | " |
| 143 | " | 100<br>45 | " | " |
| 165 | " | 100<br>25 | 10<br>0 | " |
| 243 | " | 100<br>40 | 15<br>0 | " |

(to be cont'd)



| | | | | |
|---|---|---|---|---|
| 244 | 200<br>50 | 100<br>20 | 10<br>0 | 0<br>0 |
| 245 | " | " | 15<br>0 | " |
| 252 | " | 100<br>25 | " | " |
| 262 | " | 100<br>55 | 20<br>0 | " |
| 263 | " | " | 25<br>0 | " |
| 267 | " | " | " | " |
| 269 | " | 100<br>25 | 15<br>0 | " |
| 270 | " | 100<br>50 | 20<br>0 | " |
| 282 | " | 100<br>20 | 10<br>0 | " |
| 300 | " | " | " | " |
| 301 | " | 100<br>30 | 15<br>0 | 0 |
| 302 | " | " | " | " |
| 405 | " | 100<br>35 | " | " |
| 434 | " | " | " | " |
| 441 | " | 100<br>25 | 10<br>0 | " |
| BT-1 | 40<br>10 | 0<br>0 | 30<br>0 | 100<br>40 |
| BT-4 | " | " | 20<br>0 | 100<br>30 |

| | | | | |
|---|---|---|---|---|
| BT-5 | 40<br>10 | 0<br>0 | 5<br>0 | 100<br>35 |
| BT-6 | " | " | 20<br>0 | " |

* BT-1 :

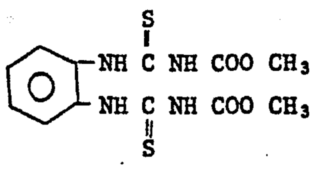

(thiophanate methyl)

BT-4 :

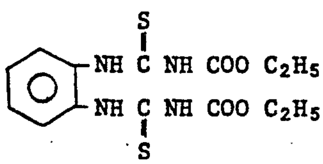

(thiophanate)

BT-5 :

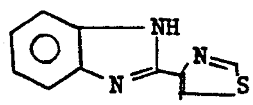

(thiabendazole)

BT-6 :

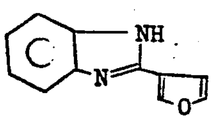

(fuberidazole)

Test 3 Cercospora leaf spot (Cercospora beticola) of beet:

A sufficient amount of an aqueous solution having a specified concentration was prepared from a wettable powder of each test compound, and sprayed over young seedling of beet (Variety: Monohil, in the 5th to the 6th leaf stage) cultivated in a porous unglazed pot having 9 cm of diameter.

The leaves were air-dried and inoculated with conidia of cercospora leaf spot (Cercospora beticola) resistant or sensitive to benzimidazole-thiophanate series compounds or a mixture thereof by means of spraying.

The inoculated young beets were placed in the high humid room at 24 - 26°C for one day and kept in the greenhouse at 23 - 28°C for 12 days.

Then, occurrence status of disease spots was investigated. In compliance with the following standard method, disease indexes, as shown below, in each treating zone were surveyed, and preventive values (%) were calculated by the following preventive value calculating formula:

| The Disease Index | | Occurrence Status of Disease Spots |
|---|---|---|
| 0 | : | Disease occurrence was not entirely identfied. |
| 0.5 | : | 3 - 5 pieces of disease spots were identfied on a leaf. |
| 1 | : | 10 - 25% of leaf surface area was affected with disease spots. |
| 2 | : | 26 - 50% of leaf surface area was affected with disease spots. |
| 3 | : | 51 - 75% of leaf surface area was affected with disease spots. |

| | | |
|---|---|---|
| 4 | : | 75% or more of leaf surface area was affected with disease spots. |

$$\text{Preventive value (\%)} = \left(1 - \frac{\text{Average disease index in treated zone}}{\text{Average disease index in untreated zone}}\right) \times 100$$

The results are shown in Table 4 (4-1 and 4-2) as follows:

Table 4

4-1 Activity of the compound

| Compound No. | Concentration of Active ingredient (ppm) | Preventive Value (%) | |
|---|---|---|---|
| | | Chemical Resistant Fungi | Chemical Sensitive Fungi |
| 8 | 400 | 100 | 0 |
| 9 | " | " | " |
| 10 | " | " | " |
| 11 | " | 97 | " |
| 13 | " | 100 | " |
| 14 | " | " | " |
| 16 | " | 81 | " |
| 18 | " | 100 | " |
| 23 | " | " | " |
| 24 | " | 81 | " |
| 26 | " | 94 | " |
| 33 | " | 100 | " |
| 34 | " | " | " |
| 35 | " | " | " |
| 36 | " | " | " |
| 37 | " | 97 | " |
| 38 | " | 100 | " |
| 39 | " | " | " |
| 40 | " | " | " |
| 41 | " | " | " |
| 44 | " | 97 | " |
| 45 | " | 100 | " |
| 46 | " | 88 | " |

(to be cont'd)



| | | | |
|---|---|---|---|
| 50 | 400 | 88 | 0 |
| 51 | " | 97 | " |
| 53 | " | 100 | " |
| 54 | " | " | " |
| 56 | " | 91 | " |
| 60 | " | 100 | " |
| 62 | " | " | " |
| 64 | " | 81 | " |
| 67 | " | " | " |
| 70 | " | " | " |
| 72 | " | 91 | " |
| 75 | " | 97 | " |
| 77 | " | 100 | " |
| 78 | " | 97 | " |
| 79 | " | 100 | " |
| 80 | " | " | " |
| 81 | " | " | " |
| 82 | " | " | " |
| 83 | " | " | " |
| 84 | " | " | " |
| 85 | " | " | " |
| 86 | " | 91 | " |
| 87 | " | 94 | " |
| 88 | " | 100 | " |
| 89 | " | " | " |
| 90 | " | 90 | " |
| 91 | " | 100 | " |
| 92 | " | 94 | " |

(to be cont'd)

| | | | |
|---|---|---|---|
| 93 | 400 | 100 | 0 |
| 94 | " | " | " |
| 95 | " | " | " |
| 96 | " | " | " |
| 97 | " | " | " |
| 99 | " | 81 | " |
| 100 | " | " | " |
| 102 | " | 100 | " |
| 104 | " | " | " |
| 108 | " | " | " |
| 109 | " | 97 | " |
| 110 | " | " | " |
| 111 | " | 100 | " |
| 112 | " | 97 | " |
| 113 | " | 100 | " |
| 114 | " | " | " |
| 115 | " | " | " |
| 116 | " | " | " |
| 117 | " | " | " |
| 118 | " | " | " |
| 119 | " | " | " |
| 120 | " | " | " |
| 121 | " | " | " |
| 122 | " | " | " |
| 125 | " | " | " |
| 126 | " | " | " |
| 127 | " | " | " |
| 128 | " | " | " |

(to be cont'd)



| | | | |
|---|---|---|---|
| 129 | 400 | 100 | 0 |
| 130 | " | " | " |
| 132 | " | 84 | " |
| 134 | " | 100 | " |
| 135 | " | " | " |
| 136 | " | 97 | " |
| 139 | " | 88 | " |
| 140 | " | 100 | " |
| 141 | " | " | " |
| 142 | " | " | " |
| 143 | " | 94 | " |
| 144 | " | 97 | " |
| 146 | " | 91 | " |
| 159 | " | " | " |
| 160 | " | 97 | " |
| 165 | " | 100 | " |
| 166 | " | " | " |
| 167 | " | 97 | " |
| 168 | " | 100 | " |
| 170 | " | " | " |
| 175 | " | " | " |
| 178 | " | " | " |
| 179 | " | " | " |
| 180 | " | " | " |
| 181 | " | " | " |
| 182 | " | " | " |
| 183 | " | " | " |
| 184 | " | " | " |

(to be cont'd)

| | | | |
|---|---|---|---|
| 199 | 400 | 100 | 0 |
| 200 | " | " | " |
| 201 | " | 97 | " |
| 202 | " | 100 | " |
| 209 | " | " | " |
| 211 | " | " | " |
| 212 | " | 88 | " |
| 214 | " | 100 | " |
| 224 | " | " | " |
| 231 | " | 94 | " |
| 232 | " | 84 | " |
| 233 | " | 91 | " |
| 235 | " | 100 | " |
| 236 | " | " | " |
| 241 | " | " | " |
| 242 | " | " | " |
| 244 | " | " | " |
| 245 | " | " | " |
| 246 | " | " | " |
| 249 | " | " | " |
| 250 | " | 97 | " |
| 252 | " | 100 | " |
| 253 | " | " | " |
| 254 | " | " | " |
| 255 | " | " | " |
| 256 | " | 94 | " |
| 267 | " | 100 | " |
| 268 | " | " | " |

| | | | |
|---|---|---|---|
| 269 | 400 | 100 | 0 |
| 270 | " | 91 | " |
| 272 | " | 100 | " |
| 279 | " | 97 | " |
| 282 | " | 81 | " |
| 283 | " | 91 | " |
| 284 | " | 100 | " |
| 285 | " | " | " |
| 286 | " | 88 | " |
| 295 | " | 100 | " |
| 296 | " | " | " |
| 298 | " | " | " |
| 300 | " | " | " |
| 302 | " | " | " |
| 304 | " | " | " |
| 305 | " | " | " |
| 309 | " | 88 | " |
| 326 | " | 94 | " |
| 333 | " | 81 | " |
| 348 | " | 100 | " |
| 350 | " | 88 | " |
| 351 | " | 84 | " |
| 352 | " | 100 | " |
| 353 | " | " | " |
| 356 | " | 78 | " |
| 365 | " | 72 | " |
| 401 | " | 97 | " |
| 403 | " | 94 | " |

| | | | |
|---|---|---|---|
| 406 | 400 | 88 | 0 |
| 407 | " | 97 | " |
| 411 | " | 100 | " |
| 412 | " | 88 | " |
| 414 | " | 100 | " |
| 415 | " | 97 | " |
| 416 | " | 100 | " |
| 417 | " | 81 | " |
| 418 | " | 100 | " |
| 421 | " | " | " |
| 422 | " | 97 | " |
| 423 | " | 94 | " |
| 424 | " | 100 | " |
| 425 | " | " | " |
| 426 | " | 94 | " |
| 428 | " | 100 | " |
| 429 | " | " | " |
| 430 | " | " | " |
| 431 | " | " | " |
| 432 | " | " | " |
| 433 | " | " | " |
| 434 | " | " | " |
| 435 | " | " | " |
| 436 | " | 97 | " |
| 437 | " | " | " |
| 438 | " | 100 | " |
| 439 | " | 91 | " |
| 440 | " | 97 | " |

| | | | |
|---|---|---|---|
| 441 | 400 | 94 | 0 |
| 442 | " | 100 | " |
| 443 | " | " | " |
| 444 | " | " | " |
| 445 | " | " | " |
| 448 | " | 91 | " |
| 449 | " | 94 | " |
| 454 | " | 97 | " |
| Comparative Compound 1* | " | 0 | 100 |
| " 2 | " | 80 | 80 |
| " 3 | " | 0 | 0 |

* Comparative Compound 1 : thiophanate methyl

" 2 : fentin hydroxide

" 3 :

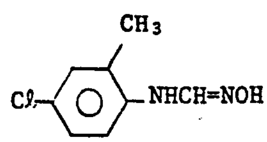

(U.S.P. 4,237,168)

Table 4

4-2 Activity of Mixed Chemicals

| Compound No. | Concentration of Active ingredient (ppm) | Preventive Value (%) | | |
|---|---|---|---|---|
| | | Chemical Resistant Fungi | Mixed Fungi | Chemical Sensitive Fungi |
| 6<br>BT-1 | 50<br>12.5 | 100 | 100 | 100 |
| 6<br>BT-2 | " | " | " | " |
| 6<br>BT-3 | " | " | " | " |
| 6<br>BT-4 | " | " | " | " |
| 7<br>BT-1 | " | " | " | " |
| 7<br>BT-2 | " | " | " | " |
| 7<br>BT-3 | " | " | " | " |
| 7<br>BT-4 | " | " | " | " |
| 19<br>BT-1 | " | " | " | " |
| 19<br>BT-2 | " | " | " | " |
| 19<br>BT-3 | " | " | " | " |
| 19<br>BT-4 | " | " | " | " |
| 29<br>BT-1 | " | " | " | " |

| | | | | |
|---|---|---|---|---|
| 29<br>BT-2 | 50<br>12.5 | 100 | 100 | 100 |
| 29<br>BT-3 | " | " | " | " |
| 29<br>BT-4 | " | " | " | " |
| 30<br>BT-1 | " | " | " | " |
| 30<br>BT-2 | " | " | " | " |
| 30<br>BT-3 | " | " | " | " |
| 30<br>BT-4 | " | " | " | " |
| 31<br>BT-1 | " | " | " | " |
| 31<br>BT-2 | " | " | " | " |
| 31<br>BT-3 | " | " | " | " |
| 31<br>BT-4 | " | " | " | " |
| 32<br>BT-1 | " | " | " | " |
| 32<br>BT-2 | " | " | " | " |
| 32<br>BT-3 | " | " | " | " |
| 32<br>BT-4 | " | " | " | " |
| 47<br>BT-1 | " | " | " | " |

(to be cont'd)



| | | | | |
|---|---|---|---|---|
| 47<br>BT-2 | 50<br>12.5 | 100 | 100 | 100 |
| 47<br>BT-3 | " | " | " | " |
| 47<br>BT-4 | " | " | " | " |
| 61<br>BT-1 | " | " | " | " |
| 61<br>BT-2 | " | " | " | " |
| 61<br>BT-3 | " | " | " | " |
| 61<br>BT-4 | " | " | " | " |
| 101<br>BT-1 | " | " | " | " |
| 101<br>BT-2 | " | " | " | " |
| 101<br>BT-3 | " | " | " | " |
| 101<br>BT-4 | " | " | " | " |
| 105<br>BT-1 | " | " | " | " |
| 105<br>BT-2 | " | " | " | " |
| 105<br>BT-3 | " | " | " | " |
| 105<br>BT-4 | " | " | " | " |
| 106<br>BT-1 | " | " | " | " |

| | | | | |
|---|---|---|---|---|
| 106<br>BT-2 | 50<br>12.5 | 100 | 100 | 100 |
| 106<br>BT-3 | " | " | " | " |
| 106<br>BT-4 | " | " | " | " |
| 107<br>BT-1 | " | " | " | " |
| 107<br>BT-2 | " | " | " | " |
| 107<br>BT-3 | " | " | " | " |
| 107<br>BT-4 | " | " | " | " |
| 123<br>BT-1 | " | " | " | " |
| 123<br>BT-2 | " | " | " | " |
| 123<br>BT-3 | " | " | " | " |
| 123<br>BT-4 | " | " | " | " |
| 124<br>BT-1 | " | " | " | " |
| 124<br>BT-2 | " | " | " | " |
| 124<br>BT-3 | " | " | " | " |
| 124<br>BT-4 | " | " | " | " |
| 147<br>BT-1 | " | " | " | " |

(to be cont'd)



| | | | | |
|---|---|---|---|---|
| 147<br>BT-2 | 50<br>12.5 | 100 | 100 | 100 |
| 147<br>BT-3 | " | " | " | " |
| 147<br>BT-4 | " | " | " | " |
| 157<br>BT-1 | " | " | " | " |
| 157<br>BT-2 | " | " | " | " |
| 157<br>BT-3 | " | " | " | " |
| 157<br>BT-4 | " | " | " | " |
| 208<br>BT-1 | " | " | " | " |
| 208<br>BT-2 | " | " | " | " |
| 208<br>BT-3 | " | " | " | " |
| 208<br>BT-4 | " | " | " | " |
| 229<br>BT-1 | " | " | " | " |
| 229<br>BT-2 | " | " | " | " |
| 229<br>BT-3 | " | " | " | " |
| 229<br>BT-4 | " | " | " | " |
| 243<br>BT-1 | " | " | " | " |

| | | | | |
|---|---|---|---|---|
| 243 BT-2 | 50 12.5 | 100 | 100 | 100 |
| 243 BT-3 | " | " | " | " |
| 243 BT-4 | " | " | " | " |
| 251 BT-1 | " | " | " | " |
| 251 BT-2 | " | " | " | " |
| 251 BT-3 | " | " | " | " |
| 251 BT-4 | " | " | " | " |
| 260 BT-1 | " | " | " | " |
| 260 BT-2 | " | " | " | " |
| 260 BT-3 | " | " | " | " |
| 260 BT-4 | " | " | " | " |
| 262 BT-1 | " | " | " | " |
| 262 BT-2 | " | " | " | " |
| 262 BT-3 | " | " | " | " |
| 262 BT-4 | " | " | " | " |
| 263 BT-1 | " | " | " | " |

(to be cont'd)



| | | | | |
|---|---|---|---|---|
| 263<br>BT-2 | 50<br>12.5 | 100 | 100 | 100 |
| 263<br>BT-3 | " | " | " | " |
| 263<br>BT-4 | " | " | " | " |
| 287<br>BT-1 | " | " | " | " |
| 287<br>BT-2 | " | " | " | " |
| 287<br>BT-3 | " | " | " | " |
| 287<br>BT-4 | " | " | " | " |
| 288<br>BT-1 | " | " | " | " |
| 288<br>BT-2 | " | " | " | " |
| 288<br>BT-3 | " | " | " | " |
| 288<br>BT-4 | " | " | " | " |
| 310<br>BT-1 | " | " | " | " |
| 310<br>BT-2 | " | " | " | " |
| 310<br>BT-3 | " | " | " | " |
| 310<br>BT-4 | " | " | " | " |
| 398<br>BT-1 | " | " | " | " |

(to be cont'd)



| | | | | |
|---|---|---|---|---|
| 398<br>BT-2 | 50<br>12.5 | 100 | 100 | 100 |
| 398<br>BT-3 | " | " | " | " |
| 398<br>BT-4 | " | " | " | " |
| 399<br>BT-1 | " | " | " | " |
| 399<br>BT-2 | " | " | " | " |
| 399<br>BT-3 | " | " | " | " |
| 399<br>BT-4 | " | " | " | " |
| 400<br>BT-1 | " | " | " | " |
| 400<br>BT-2 | " | " | " | " |
| 400<br>BT-3 | " | " | " | " |
| 400<br>BT-4 | " | " | " | " |
| 409<br>BT-1 | " | " | " | " |
| 409<br>BT-2 | " | " | " | " |
| 409<br>BT-3 | " | " | " | " |
| 409<br>BT-4 | " | " | " | " |
| 410<br>BT-1 | " | " | " | " |

(to be cont'd)

| | | | | |
|---|---|---|---|---|
| 410<br>BT-2 | 50<br>12.5 | 100 | 100 | 100 |
| 410<br>BT-3 | " | " | " | " |
| 410<br>BT-4 | " | " | " | " |
| 419<br>BT-1 | " | " | " | " |
| 419<br>BT-2 | " | " | " | " |
| 419<br>BT-3 | " | " | " | " |
| 419<br>BT-4 | " | " | " | " |
| 420<br>BT-1 | " | " | " | " |
| 420<br>BT-2 | " | " | " | " |
| 420<br>BT-3 | " | " | " | " |
| 420<br>BT-4 | " | " | " | " |
| 6 | 400<br>50 | 100<br>20 | 10<br>0 | 0<br>0 |
| 7 | " | 100<br>40 | 20<br>0 | " |
| 19 | " | 100<br>30 | 15<br>0 | " |
| 29 | " | " | " | " |
| 30 | " | 100<br>40 | 30<br>0 | " |

| | | | | |
|---|---|---|---|---|
| 31 | 400<br>50 | 100<br>40 | 25<br>0 | 0<br>0 |
| 32 | " | " | " | " |
| 47 | " | 100<br>35 | 20<br>0 | " |
| 61 | " | 100<br>30 | 15<br>0 | " |
| 101 | " | 100<br>40 | 10<br>0 | " |
| 105 | " | 100<br>50 | 25<br>0 | " |
| 106 | " | 100<br>35 | 5<br>0 | " |
| 107 | " | 100<br>20 | 20<br>0 | " |
| 123 | " | " | " | " |
| 124 | " | 100<br>25 | " | " |
| 147 | " | " | " | " |
| 157 | " | 100<br>45 | " | " |
| 208 | " | 95<br>45 | 15<br>0 | " |
| 229 | " | 100<br>35 | 20<br>0 | " |
| 243 | " | 100<br>20 | 15<br>0 | " |
| 251 | " | 100<br>10 | " | " |
| 260 | " | 100<br>35 | 10<br>0 | " |

| | | | | |
|---|---|---|---|---|
| 262 | 400<br>50 | 100<br>35 | 10<br>0 | 0<br>0 |
| 263 | " | 100<br>45 | " | " |
| 287 | " | " | " | " |
| 288 | " | 100<br>15 | " | " |
| 310 | " | 100<br>30 | 15<br>0 | " |
| 398 | " | 100<br>20 | " | " |
| 399 | " | " | 20<br>0 | " |
| 400 | " | 100<br>50 | " | " |
| 409 | " | 100<br>20 | 10<br>0 | " |
| 410 | " | 100<br>35 | " | " |
| 419 | " | 100<br>45 | " | " |
| 420 | " | 100<br>40 | " | " |
| BT-1* | 50<br>12.5 | 0<br>0 | 25<br>0 | 100<br>40 |
| BT-2 | " | " | 35<br>0 | 100<br>45 |
| BT-3 | " | " | " | " |
| BT-4 | " | " | 15<br>0 | 100<br>20 |

* BT-1 to BT-4 : The same as those in Test 1 or Test 2.

Test 4 Scab (Venturia inaequalis) of apple:

An aqueous solution having a specified concentration prepared from each test compound was sprayed over the young seedling of apple in the 3th to 4th leaf stage (Variety: Kokko) which was cultivated in a porous unglazed pot.

The seedling was air-dried and inoculated with conidia of scab (Venturia inaequalis) resistant or sensitive to benzimidazole-thiophanate series compounds.

Each inoculated seedling was separately placed in the humid room at 16°C and then kept in the greenhouse at 15 - 20°C, and thereby, the disease was made to occur.

After two weeks, the disease occurrence degrees for each leaf of the inoculated seedling was investigated by the following investigating standard, and the preventive value (%) for treated zone was calculated by the following calculation formula:

The investigating standard:

| Disease index | 0 | 1 | 2 | 3 | 4 | 5 | 6 |
|---|---|---|---|---|---|---|---|
| Leaf surface area affected with disease spots (%) | healthy | 10% or less | 11∿20% | 21∿30% | 31∿40% | 41∿50% | 51∿60% |

(to be cont')

| 7 | 8 | 9 | 10 |
|---|---|---|---|
| 61∿70% | 71∿80% | 81∿90% | 91∿100% |

$$\text{Preventive value (\%)} = \left(1 - \frac{\text{Average disease index in treated zone}}{\text{Average disease index in untreated zone}}\right) \times 100$$

The results are shown in Table 5 (5-1 and 5-2) as follows:

Table 5

5-1 Activity of the compound

| Compound No. | Concentration of Active ingredient (ppm) | Preventive Value (%) | |
|---|---|---|---|
| | | Chemical Resistant Fungi | Chemical Sensitive Fungi |
| 6 | 200 | 100 | 0 |
| 8 | " | " | " |
| 9 | " | " | " |
| 13 | " | " | " |
| 14 | " | " | " |
| 18 | " | " | " |
| 19 | " | " | " |
| 23 | " | " | " |
| 29 | " | " | " |
| 31 | " | " | 7 |
| 33 | " | " | 0 |
| 34 | " | " | 3 |
| 35 | " | " | 7 |
| 36 | " | " | 0 |
| 37 | " | " | " |
| 38 | " | " | 20 |
| 39 | " | " | 0 |
| 45 | " | " | 10 |
| 50 | " | 85 | 0 |
| 54 | " | 100 | " |
| 56 | " | " | " |
| 60 | " | " | 24 |

(to be cont'd)



| | | | |
|---|---|---|---|
| 61 | 200 | 100 | 20 |
| 62 | " | " | 15 |
| 77 | " | " | 0 |
| 78 | " | " | 7 |
| 79 | " | " | 0 |
| 80 | " | " | " |
| 81 | " | " | " |
| 83 | " | " | 20 |
| 85 | " | " | 10 |
| 88 | " | " | 14 |
| 89 | " | " | 0 |
| 90 | " | " | " |
| 91 | " | " | 7 |
| 92 | " | " | 0 |
| 93 | " | " | " |
| 94 | " | " | 7 |
| 96 | " | " | 0 |
| 101 | " | " | " |
| 104 | " | " | " |
| 105 | " | " | 14 |
| 106 | " | " | 0 |
| 107 | " | " | " |
| 109 | " | " | 10 |
| 111 | " | " | 0 |
| 112 | " | " | 20 |
| 113 | " | " | " |
| 114 | " | " | 25 |
| 115 | " | " | " |

| | | | |
|---|---|---|---|
| 116 | 200 | 100 | 0 |
| 117 | " | " | " |
| 118 | " | " | 20 |
| 119 | " | " | 7 |
| 120 | " | " | 0 |
| 121 | " | " | 20 |
| 123 | " | " | 0 |
| 124 | " | " | " |
| 125 | " | " | 25 |
| 126 | " | " | 20 |
| 127 | " | " | 0 |
| 129 | " | " | " |
| 134 | " | " | " |
| 135 | " | " | " |
| 139 | " | " | " |
| 140 | " | " | 25 |
| 141 | " | " | 20 |
| 142 | " | " | 25 |
| 143 | " | " | 0 |
| 147 | " | " | 20 |
| 160 | " | " | 0 |
| 165 | " | " | " |
| 200 | " | " | 20 |
| 229 | " | " | 0 |
| 231 | " | " | " |
| 241 | " | " | 24 |
| 243 | " | " | 7 |
| 244 | " | " | 0 |

| | | | |
|---|---|---|---|
| 245 | 200 | 100 | 0 |
| 246 | " | " | " |
| 249 | " | " | " |
| 250 | " | " | " |
| 251 | " | " | " |
| 252 | " | " | 14 |
| 253 | " | " | 17 |
| 254 | " | " | 0 |
| 255 | " | " | " |
| 267 | " | " | " |
| 268 | " | " | " |
| 269 | " | " | 10 |
| 270 | " | " | " |
| 282 | " | " | 14 |
| 285 | " | " | 0 |
| 286 | " | " | " |
| 287 | " | " | " |
| 295 | " | " | " |
| 296 | " | " | " |
| 298 | " | " | " |
| 302 | " | " | " |
| 304 | " | " | " |
| 305 | " | " | 3 |
| 309 | " | " | 14 |
| 310 | " | " | 25 |
| 400 | " | " | 0 |
| 406 | " | " | " |
| 409 | " | " | " |

(to be cont'd)



| | | | |
|---|---|---|---|
| 410 | 200 | 100 | 0 |
| 411 | " | " | " |
| 414 | " | " | " |
| 415 | " | " | " |
| 416 | " | " | " |
| 417 | " | " | 20 |
| 418 | " | " | " |
| 420 | " | " | 0 |
| 421 | " | " | " |
| 422 | " | " | " |
| 423 | " | " | " |
| 424 | " | " | " |
| 428 | " | " | " |
| 429 | " | " | " |
| 430 | " | " | 10 |
| 432 | " | " | 0 |
| 433 | " | " | 14 |
| 434 | " | " | 0 |
| 435 | " | " | " |
| 440 | " | " | " |
| 441 | " | " | 20 |
| 442 | " | " | 0 |
| 443 | " | " | " |
| 445 | " | " | " |
| 454 | " | " | " |
| Comparative Compound 1* | " | 0 | 100 |
| " 2 | " | 80 | 80 |

(to be cont'd)

| | | | |
|---|---|---|---|
| Comparative Compound 3 | 200 | 0 | 0 |

* Comparative Compound 1 : thiophanate methyl

" 2 : captan

" 3 :

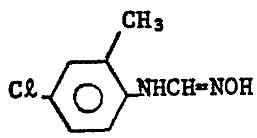

(U.S.P. 4,237,168)

Table 5

5-2 Activity of Mixed Chemicals

| Compound No. | Concentration of Active ingredient (ppm) | Preventive Value (%) | | |
|---|---|---|---|---|
| | | Chemical Resistant Fungi | Mixed Fungi | Chemical Sensitive Fungi |
| 7<br>BT-1 | 50<br>50 | 100 | 100 | 100 |
| 7<br>BT-2 | " | " | " | " |
| 10<br>BT-1 | " | " | " | " |
| 10<br>BT-2 | " | " | " | " |
| 30<br>BT-1 | " | " | " | " |
| 30<br>BT-2 | " | " | " | " |
| 32<br>BT-1 | " | " | " | " |
| 32<br>BT-2 | " | " | " | " |
| 47<br>BT-1 | " | " | " | " |
| 47<br>BT-2 | " | " | " | " |
| 95<br>BT-1 | " | " | " | " |
| 95<br>BT-2 | " | " | " | " |

(to be cont'd)



| | | | | |
|---|---|---|---|---|
| 97<br>BT-1 | 50<br>50 | 100 | 100 | 100 |
| 97<br>BT-2 | " | " | " | " |
| 110<br>BT-1 | " | " | " | " |
| 110<br>BT-2 | " | " | " | " |
| 259<br>BT-1 | " | " | " | " |
| 259<br>BT-2 | " | " | " | " |
| 260<br>BT-1 | " | " | " | " |
| 260<br>BT-2 | " | " | " | " |
| 262<br>BT-1 | " | " | " | " |
| 262<br>BT-2 | " | " | " | " |
| 263<br>BT-1 | " | " | " | " |
| 263<br>BT-2 | " | " | " | " |
| 407<br>BT-1 | " | " | " | " |
| 407<br>BT-2 | " | " | " | " |
| 419<br>BT-1 | " | " | " | " |
| 419<br>BT-2 | " | " | " | " |

| | | | | |
|---|---|---|---|---|
| 425<br>BT-1 | 50<br>50 | 100 | 100 | 100 |
| 425<br>BT-2 | " | " | " | " |
| 7 | 200<br>50 | 100<br>30 | 10<br>0 | 0<br>0 |
| 10 | " | " | 15<br>0 | " |
| 30 | " | 100<br>40 | 10<br>0 | " |
| 32 | " | 100<br>35 | " | " |
| 47 | " | 100<br>25 | 15<br>0 | " |
| 95 | " | 100<br>30 | 10<br>0 | 20<br>0 |
| 97 | " | " | 0<br>0 | 5<br>0 |
| 110 | " | 100<br>40 | 20<br>0 | 0<br>0 |
| 259 | " | 100<br>25 | 10<br>0 | " |
| 260 | " | 100<br>40 | 20<br>0 | 14<br>0 |
| 262 | " | 100<br>30 | " | 7<br>0 |
| 263 | " | 100<br>40 | 15<br>0 | 0<br>0 |
| 407 | " | 100<br>25 | 10<br>0 | " |

| | | | | |
|---|---|---|---|---|
| 419 | 200<br>50 | 100<br>30 | 0<br>0 | 0<br>0 |
| 425 | " | " | 10<br>0 | " |
| BT-1* | " | 0<br>0 | 25<br>0 | 100<br>60 |
| BT-2 | " | " | 30<br>0 | 100<br>65 |

* BT-1 and BT-2 : The same as those shown in Test 1

Test 5 Downy mildew (Pseudoperonospora cubensis) of cucumber:

An aqueous solution having a specified concentration which was prepared from the wettable powder of each test compound was sprayed over the young seedling of cucumber (Variety: Sagami hanjiro) cultiivated for about 3 weeks.

The seedling was air-dried and inoculated with a liquid suspension containing the zoosporangium of Pseudoperonospora cubensis collected from the cucumber leaf affected by downy mildew by means of spraying.

The seedling was placed in the inoculating box having 100% of relative humidity at 25°C.

On the second day after the inoculation, the plant was transferred to the room at a temperature of 23 - 28°C.

On the 7th day after the inoculation, the disease occurrence degree of each cucumber leaf was investigated in compliance with the following standard:

Investigating standard:

| Disease index | 0 | 0.5 | 1 | 2 | 3 | 4 |
|---|---|---|---|---|---|---|
| Surface area of leaf affected with disease lesion | healthy | status of disease lesion is fine | 25% or less | 50% or less | 75% or less | 75% or more |

The preventive value in the treated zone was calculated by the following calculating formula:

$$\text{Preventive Value (\%)} = \left(1 - \frac{\text{Average disease index in treated zone}}{\text{Average disease index in untreated zone}}\right) \times 100$$

The results are shown in Table 6 as follows:

Table 6

| Compound No. | Concentration of Active ingredient (ppm) | Preventive Value (%) |
|---|---|---|
| 4 | 400 | 87 |
| 14 | " | 81 |
| 34 | " | 94 |
| 51 | " | 87 |
| 52 | " | " |
| 53 | " | " |
| 62 | " | 81 |
| 77 | " | 94 |
| 80 | " | " |
| 83 | " | 81 |
| 94 | " | " |
| 117 | " | " |
| 125 | " | 94 |
| 126 | " | " |
| 143 | " | 100 |
| 147 | " | 87 |
| 148 | " | " |
| 217 | " | " |
| 218 | " | 81 |
| 227 | " | " |
| 228 | " | 87 |
| 231 | " | 81 |
| 311 | " | 87 |

(to be cont'd) 

| | | |
|---|---|---|
| 402 | 400 | 100 |
| 431 | " | 87 |
| 441 | " | " |
| Comparative Compound 1* | " | 84 |
| " 2 | " | 81 |

* Comparative Compound 1 : chlorotharonil

" 2 : zineb

Test 6 Insecticidal activity against Armyworm:

An emulsifiable concentrate or a wettable powder (in case of metal salt complex of compound) formulated according to the aforesaid Examples was diluted with water to obtain a solution or emulsion of 500 ppm in concentration of test compound. A leaf of corn was immersed in the liquid formulation for 30 seconds and air-dried. The treated leaf was put in a petri dish enclosing 5 third instar larvae of armyworm, and the petri dish was covered with a sheet of glass. The petri dish was placed in a room kept at 25°C and 65% relative humidity, and the mortality was investigated after 120 hours. The results obtained in two replicates are shown in Table 7.

Table 7

| Compound No. | Insecticidal activity (%) |
|---|---|
| 14 | 100 |
| 30 | " |
| 31 | " |
| 33 | " |
| 34 | " |
| 35 | " |
| 43 | " |
| 56 | " |
| 60 | " |
| 61 | " |
| 62 | " |
| 80 | " |
| 86 | " |

(to be cont'd)

| | |
|---|---|
| 87 | 100 |
| 89 | " |
| 90 | " |
| 92 | " |
| 93 | " |
| 94 | " |
| 95 | " |
| 96 | " |
| 109 | " |
| 110 | " |
| 114 | " |
| 119 | " |
| 120 | " |
| 122 | " |
| 224 | " |
| 232 | " |
| 242 | " |
| 243 | " |
| 250 | " |
| 253 | " |
| 254 | " |
| 255 | " |
| 267 | " |
| 268 | " |
| 301 | " |
| 416 | " |
| 418 | " |

(to be cont')

| | |
|---|---|
| 419 | 100 |
| 420 | " |
| 421 | " |
| 422 | " |
| 423 | " |
| 425 | " |
| 432 | " |
| 441 | " |
| 443 | " |
| Comparative Compound A* | 40 |

* Comparative Compound A :

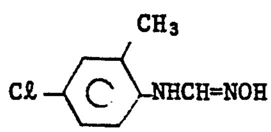

(U.S.P. 4,237,168)

Cotton aphid

Test 7 Insecticidal activity against cotton aphid:

A wettable powder of test compound formulated according to the aforesaid Examples was diluted with water to obtain a solution or suspension of 500 a.i.ppm (ppm of active ingredient).

Cotton aphids inoculated on a young cucumber plant in a pot were treated by spraying said aqueous solution of test compound and kept in a room at 25°C and 65% relative humidity. Aphicidal activity was assessed at seven days after treatment. Control efficacy (%) was calculated by the formula of (1-B/A) X 100, wherein A means the increasing rate (number of aphids at 7 days after treatment per number of aphids before treatment) in the untreated pot and B means the increasing rate in the treated pot.

The results are shown in Table 8.

Table 8

| Compound No. | Control Efficacy (%) |
|---|---|
| 1 | 100 |
| 4 | " |
| 34 | " |
| 36 | 99 |
| 46 | 100 |
| 47 | " |
| 49 | " |
| 50 | " |
| 51 | " |
| 52 | " |

(to be cont')

| | |
|---|---|
| 53 | 100 |
| 55 | 95 |
| 64 | 94 |
| 89 | 100 |
| 90 | 91 |
| 94 | 94 |
| 101 | 100 |
| 114 | 96 |
| 128 | 90 |
| 142 | 100 |
| 147 | " |
| 148 | " |
| 165 | 93 |
| 216 | 100 |
| 217 | " |
| 218 | " |
| 220 | " |
| 222 | 90 |
| 223 | 100 |
| 227 | " |
| 228 | " |
| 231 | " |
| 294 | " |
| 305 | " |
| 307 | 92 |
| 309 | 100 |

(to be cont'd)

| | |
|---|---|
| 311 | 100 |
| 318 | " |
| 319 | " |
| 324 | " |
| 325 | " |
| 329 | " |
| 339 | " |
| 343 | " |
| 362 | " |
| 411 | " |
| Comparative Compound A* | 84 |

* Comparative Compound A : the same compound as shown in Test 6

Green rice leafhopper

Test 8 Insecticidal activity against green rice leafhopper:

An emulsifiable concentrate or a wettable powder (in case of metal salt complex of compound) formulated according to the aforesaid Examples was diluted with water to obtain a solution or suspension of 125 a.i.ppm.

Young rice plants laid eggs by adult females of green rice leafhopper were dipped into said aqueous solution and were kept in the room at 25°C and 65% relative humidity.

The number of survival larval was counted at thirteen days after treatment.

The insecticidal activity (%) was culculated by the formula of (1-B/A) X 100, wherein A means the number of larvae in the untreated pot and B means the number of larvae in the treated pot.

The results are shown in Table 9.

Table 9

| Compound No. | Insecticidal activity (%) |
|---|---|
| 5 | 100 |
| 6 | " |
| 13 | " |
| 15 | " |
| 19 | " |
| 35 | " |
| 40 | " |

(to be cont'd)

| | |
|---|---|
| 43 | 100 |
| 44 | " |
| 59 | " |
| 61 | " |
| 75 | " |
| 83 | " |
| 85 | " |
| 91 | " |
| 109 | " |
| 111 | " |
| 116 | " |
| 118 | " |
| 119 | " |
| 120 | " |
| 124 | " |
| 125 | " |
| 126 | " |
| 129 | " |
| 135 | " |
| 136 | " |
| 137 | " |
| 141 | " |
| 143 | " |
| 145 | " |
| 149 | " |
| 209 | " |

(to be cont'd)

| | |
|---|---|
| 212 | 100 |
| 219 | " |
| 224 | " |
| 229 | " |
| 230 | " |
| 241 | " |
| 245 | " |
| 255 | " |
| 256 | " |
| 270 | " |
| 296 | " |
| 297 | " |
| 300 | " |
| 310 | " |
| 317 | " |
| 320 | " |
| 321 | " |
| 325 | " |
| 327 | " |
| 328 | " |
| 334 | " |
| 335 | " |
| 338 | " |
| 340 | " |
| 343 | " |
| 344 | " |

(to be cont'd)

| | |
|---|---|
| 345 | 100 |
| 347 | " |
| 355 | " |
| 357 | " |
| 358 | " |
| 363 | " |
| 366 | " |
| 401 | " |
| 404 | " |
| 412 | " |
| 416 | " |
| 419 | " |
| 420 | " |
| 432 | " |
| 436 | " |
| 437 | " |
| 438 | " |
| 441 | " |
| 447 | " |
| 448 | " |
| 449 | " |
| 450 | " |
| 451 | " |
| Comparative Compound A* | 81 |

*Comparative Compound A : The same compound as shown in Test 6

Test 9 Two-spotted spider mite:

The primary leaves of kidney beans planted in pots were infested respectively with 30 adult females of the two-spotted spider mite. The leaves were sprayed until dew moist with an aqueous emulsion prepared with the emulsifiable concentrate of Example and containing 125 ppm of active ingredient. After 3 days of the ovipositing period, mites survived as well as killed were removed from the leaves. On the 11th day, the degree of destruction which was shown as a percentage of (1 - B/A) x 100, wherein A means the number of mites developed from eggs on untreated leaves and B means the number of mites developed from eggs on treated leaves was investigated. The result are as shown in the following Table 10.

Table 10

| Compound No. | Degree of Destruction (%) |
|---|---|
| 4 | 100 |
| 46 | " |
| 47 | " |
| 49 | " |
| 51 | 95 |
| 52 | 100 |
| 53 | " |
| 147 | " |
| 148 | " |
| 216 | " |
| 217 | " |
| 218 | " |

(to be cont'd)

| | |
|---|---|
| 227 | 100 |
| 228 | " |
| 311 | " |
| 362 | " |
| Comparative Compound A* | 81 |

* Comparative Compound A :  The same compound as shown in Test 6

What we claim is:

1. A compound having the formula

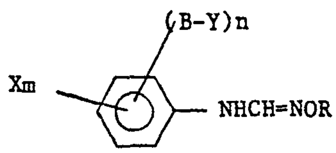

[I]

wherein X represents same or different substituent(s) selected from a group consisting of halogen, nitro,. cyano, formyl, $C_{2\sim 8}$ alkylcarbonyl, carboxy, $C_{2\sim 8}$ alkoxycarbonyl, $C_{3\sim 8}$ alkenyloxycarbonyl, $C_{3\sim 8}$ alkynyloxycarboxyl, carbamoyl, $C_{2\sim 6}$ alkylcarbamoyl, hetrocyclic radical containing oxygen, and satulated or unsatulated $C_{1\sim 6}$ hydrocarbon radicals which may be substituted by cyano, hydroxy, halogen, $C_{1\sim 6}$ alkoxy, $C_{1\sim 6}$ alkoxy substituted by $C_{1\sim 6}$ alkoxy, $C_{2\sim 6}$ alkenyloxy, $C_{2\sim 6}$ alkynyloxy, $C_{3\sim 8}$ alkynyloxycarbonyloxy, $C_{1\sim 6}$ alkylthio, $C_{1\sim 6}$ alkylsulfinyl, $C_{1\sim 6}$ alkylsulfonyl, amino substituted by $C_{1\sim 6}$ alkyl, hydroxyimino, $C_{1\sim 6}$ alkoxyimino or $C_{2\sim 8}$ alkoxycarbonyl; and

-B- represents -O-, -S-, -SO-, $-SO_2-$ or $-\overset{R'}{\overset{|}{N}}-$ (R' is hydrogen or $C_{1\sim 6}$ alkyl); and

Y represents hydrogen or same or different substituent(s) selected from a group consisting of satulated or unsaturated $C_{1\sim 6}$ hydrocarbon radicals which may be substituted by halogen, cyano, $C_{3\sim 6}$ cycloalkyl, $C_{2\sim 8}$ alkylcarbonyloxy, $C_{2\sim 8}$ alkylcarbonyl, $C_{2\sim 8}$ alkoxycarbonyl, hydroxy, $C_{1\sim 6}$ alkoxy, $C_{1\sim 6}$ alkylthio, ureido or heterocyclic radical containing oxygen; and

a part of $\leftarrow$ B-Y)n represents bi-substitutive modified radical $\leftarrow$ B-Y $\rightarrow$ selected from a group consisting of $C_{1\sim 3}$ alkylenedioxy

which may be substituted by $C_1\sim_6$ alkoxy, $-O-(CH_2)_\ell-O(CH_2)_{\ell'}-$ and $-O-(CH_2)_\ell-O-CO-$(each of $\ell$ and $\ell'$ is a integer from 1 to 3); and

each of m and n represents an integer from 0 to 5 with the proviso that $0 \leq m+n \leq 5$ and the substituent shown above takes "one", except the bi-substitutive modified -B-Y type radical $\leftarrow$ B-Y $\rightarrow$ which takes "two"; and

R represents a substituent selected from a group consisting of satulated or unsatulated $C_1\sim_{18}$ hydrocarbon radicals which may be substituted by halogen, cyano, $C_1\sim_6$ alkoxy, $C_1\sim_6$ alkylthio, $C_1\sim_6$ alkoxycarbonyl; and

with the proviso that "C number $\sim$ number" represents the range of total carbon number of the substituent or radical directly following thereto;

or a salt thereof with an organic or inorganic acid; or a complex thereof with a metal salt.

2. A compound in accordance with claim 1, wherein one or more of -B-Y substituent(s) exist on the phenyl radical of the formula (I).

3. A compound in accordance with claim 2, wherein -B- represents -O-.

4. A compound in accordance with claim 3, wherein Y represents sutulated or unsatulated $C_1\sim_6$ hydrocarbon radical which may be substituted by halogen.

5. A compound in accordance with claim 2, wherein one of -B-Y substituent(s) is located on 3 or 4 position on the phenyl radical of the formula [I].

6. A fungicidal composition comprising on inert carrier and an effective amount of a compound of claim 1.

7. A fungicidal composition comprising an inert carrier and an effective amount of a compound of claim 2.

8. A fungicidal composition comprising an inert carrier and an effective amount of a compound of claim 3.

9. A fungicidal composition comprising an inert carrier and an effective amount of a compound of claim 4.

10. A fungicidal composition comprising an inert carrier and an effective amount of a compound of claim 5.

11. A process for the control of fungi comprising applying to locus or loci of plants an effective amount of a compound of claim 1.

12. A process for the control of fungi comprising applying to locus or loci of plants an effective amount of a compound of claim 2.

13. A process for the control of fungi comprising applying to locus or loci of plants an effective amount of a compound of claim 3.

14. A process for the control of fungi comprising applying to locus or loci of plants an effective amount of a compound of claim 4.

15. A process for the control of fungi comprising applying to locus or loci of plants an effective amount of a compound of claim 5.

16. A fungicidal composition comprising an inert carrier and an effective amount of a mixture of a compound of claim 2 and a compound selected from a group consisting of benzimidazol-thiophanate series fungicidal compounds.

17. A fungicidal composition comprising an inert carrier and an effective amount of a mixture of a compound of claim 3 and a compound selected from a group consisting of benzimidazol-thiophanate series fungicidal compounds.

18. A fungicidal composition comprising an inert carrier and an effective amount of a mixture of a compound of claim 4 and a compound selected from a group consisting of benzimidazol-thiophanate series fungicidal compounds.

19. A fungicidal composition comprising an inert carrier and an effective amount of a mixture of a compound of claim 5 and a compound selected from a group consisting of benzimidazol-thiophanate series fungicidal compounds.

20. A process for the control of fungi comprising applying to locus or loci of plants an effective amount of a mixture of a compound of claim 2 and a compound selected from a group consisting of benzimidazol-thiophanate series fungicidal compounds.

21. A process for the control of fungi comprising applying to locus or loci of plants an effective amount of a mixture of a compound of claim 3 and a compound selected from a group consisting of benzimidazol-thiophanate series fungicidal compounds.

22. A process for the control of fungi comprising applying to locus or loci of plants an effective amount of a mixture of a compound of claim 4 and a compound selected from a group consisting of benzimidazol-thiophanate series fungicidal compounds.

23. A process for the control of fungi comprising applying to locus or loci of plants an effective amount of a mixture of a compound of claim 5 and a compound selected from a group consisting of benzimidazol-thiophanate series fungicidal compounds.

24. An insecticidal composition comprising an inert carrier and an effective amount of a compound of claim 1.

25. A process for the control of insects comprising applying to locus or loci of plants an effective amount of a compound of claim 1.

26. A process for the preparation of a compound having the formula

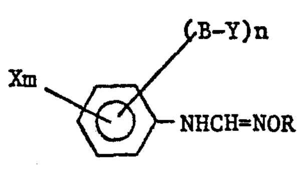

[I]

which comprises reacting a compound the formula

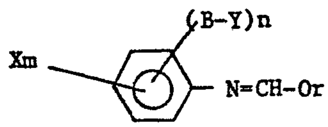

[II]

with a compound the formula

$RONH_2$ [III]

wherein X represents same or different substituent(s) selected from a group consisting of halogen, nitro, cyano, formyl, $C_{2\sim8}$ alkylcarbonyl, carboxy, $C_{2\sim8}$ alkoxycarbonyl, $C_{3\sim8}$ alkenyloxycarbonyl, $C_{3\sim8}$ alkynyloxycarboxyl, carbamoyl, $C_{2\sim6}$ alkylcarbamoyl, hetrocyclic radical containing oxygen, and satulated or unsatulated $C_{1\sim6}$ hydrocarbon radicals which may be substituted by cyano, hydroxy, halogen, $C_{1\sim6}$ alkoxy, $C_{1\sim6}$ alkoxy substituted by $C_{1\sim6}$ alkoxy, $C_{2\sim6}$ alkenyloxy, $C_{2\sim6}$ alkynyloxy, $C_{3\sim8}$ alkynyloxycarbonyloxy, $C_{1\sim6}$ alkylthio, $C_{1\sim6}$ alkylsulfinyl, $C_{1\sim6}$ alkylsulfonyl, amino substituted by $C_{1\sim6}$ alkyl, hydroxyimino, $C_{1\sim6}$ alkoxyimino or $C_{2\sim8}$ alkoxycarbonyl; and

-B- represents -O-, -S-, -SO-, $-SO_2-$ or $-\overset{R'}{\overset{|}{N}}-$ (R' is hydrogen or $C_{1\sim6}$ alkyl); and

Y represents hydrogen or same or different substituent(s) selected from a group consisting of satulated or unsaturated $C_{1\sim6}$ hydrocarbon radicals which may be substituted by halogen, cyano, $C_{3\sim6}$ cycloalkyl, $C_{2\sim8}$ alkylcarbonyloxy, $C_{2\sim8}$ alkylcarbonyl, $C_{2\sim8}$ alkoxycarbonyl, hydroxy, $C_{1\sim6}$ alkoxy, $C_{1\sim6}$ alkylthio, ureido or heterocyclic radical containing oxygen; and

a part of $-(B-Y)n$ represents bi-substitutive modified radical $-(B-Y)-$ selected from a group consisting of $C_{1\sim3}$ alkylenedioxy which may be substituted by $C_{1\sim6}$ alkoxy, $-O-(CH_2)_{\ell}-O(CH_2)_{\ell'}-$ and $-O-(CH_2)_{\ell}-O-CO-$(each of $\ell$ and $\ell'$ is a integer from 1 to 3); and

each of m and n represents an integer from 0 to 5 with the proviso that $0 \leq m+n \leq 5$ and the substituent shown above takes "one", except the bi-substitutive modified -B-Y type radical $-(B-Y)-$ which takes "two"; and

R represents a substituent selected from a group consisting of satulated or unsatulated $C_{1\sim18}$ hydrocarbon radicals which may be substituted by halogen, cyano, $C_{1\sim6}$ alkoxy, $C_{1\sim6}$ alkylthio, $C_{1\sim6}$ alkoxycarbonyl; and

r is $C_{1\sim4}$ alkyl; and

with the proviso that "C number ∿ number" represents the range of total carbon number of the substituent or radical directly following thereto.

27. A process for the preparation of a compound having the formula

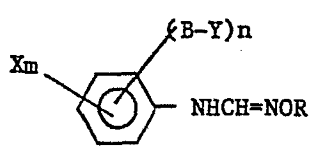

[I]

which comprises reacting a compound having the formula

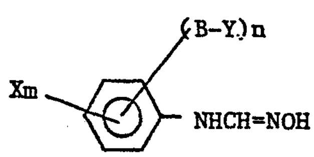

[V]

with a compound having a formula selected from a group consisting of R-Hal [VI], $(RO)_2SO_2$ [VII] and $ROSO_2r'$ [VIII]

wherein X represents same or different substituent(s) selected from a group consisting of halogen, nitro, cyano, formyl, $C_2\sim_8$ alkylcarbonyl, carboxy, $C_2\sim_8$ alkoxycarbonyl, $C_3\sim_8$ alkenyloxycarbonyl, $C_3\sim_8$ alkynyloxycarboxyl, carbamoyl, $C_2\sim_6$ alkylcarbamoyl, hetrocyclic radical containing oxygen, and satulated or unsatulated $C_1\sim_6$ - hydrocarbon radicals which may be substituted by cyano, hydroxy, halogen, $C_1\sim_6$ alkoxy, $C_1\sim_6$ alkoxy substituted by $C_1\sim_6$ alkoxy, $C_2\sim_6$ alkenyloxy, $C_2\sim_6$ alkynyloxy, $C_3\sim_8$ alkynyloxycarbonyloxy, $C_1\sim_6$ alkylthio, $C_1\sim_6$ alkylsulfinyl, $C_1\sim_6$ alkylsulfonyl, amino substituted by $C_1\sim_6$ alkyl, hydroxyimino, $C_1\sim_6$ alkoxyimino or $C_2\sim_8$ alkoxycarbonyl; and

-B- represents -O-, -S-, -SO-, $-SO_2-$ or $-\overset{R'}{\overset{|}{N}}-$ (R' is hydrogen or $C_1\sim_6$ alkyl); and

Y represents hydrogen or same or different substituent(s) selected from a group consisting of satulated or unsaturated $C_1\sim_6$ hydrocarbon radicals which may be substituted by halogen, cyano, $C_3\sim_6$ cycloalkyl, $C_2\sim_8$ alkylcarbonyloxy, $C_2\sim_8$ alkylcarbonyl, $C_2\sim_8$ alkoxycarbonyl, hydroxy, $C_1\sim_6$ alkoxy, $C_1\sim_6$ alkylthio, ureido or heterocyclic radical containing oxygen; and

a part of $(B-Y)_n$ represents bi-substitutive modified radical $(B-Y)$ selected from a group consisting of $C_1\sim_3$ alkylenedioxy which may be substituted by $C_1\sim_6$ alkoxy, $-O-(CH_2)_\ell-O(CH_2)_{\ell'}-$ and $-O-(CH_2)_\ell-O-CO-$(each of $\ell$ and $\ell'$ is a integer from 1 to 3); and

each of m and n represents an integer from 0 to 5 with the proviso that $0 \leq m+n \leq 5$ and the substituent shown above takes "one", except the bi-substitutive modified -B-Y type radical -(B-Y)- which takes "two"; and

R represents a substituent selected from a group consisting of satulated or unsatulated $C_{1\sim18}$ hydrocarbon radicals which may be substituted by halogen, cyano, $C_{1\sim6}$ alkoxy, $C_{1\sim6}$ alkylthio, $C_{1\sim6}$ alkoxycarbonyl;

Haℓ is halogen; and

r' is $C_{1\sim4}$ alkyl or phenyl which may be substituted by methyl; and

with the proviso that "C number ∿ number" represents the range of total carbon number of the substituent or radical directly following thereto.

28. A process for the preparation of a compound having the formula

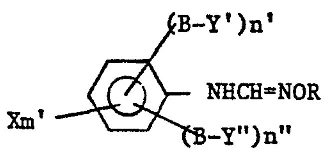

[I]'.

which comprises reacting a compound having the formula

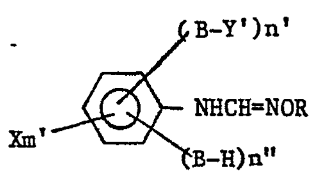

[IX]

with a compound having a formula selected from a group consisting of Y"-Hal (Y"O)$_2SO_2$ and Y"OSO$_2$r'

wherein X represents same or different substituent(s) selected from a group consisting of halogen, nitro, cyano, formyl, $C_2$∿$_8$ alkylcarbonyl, carboxy, $C_2$∿$_8$ alkoxycarbonyl, $C_3$∿$_8$ alkenyloxycarbonyl, $C_3$∿$_8$ alkynyloxycarboxyl, carbamoyl, $C_2$∿$_6$ alkylcarbamoyl, hetrocyclic radical containing oxygen, and satulated or unsatulated $C_1$∿$_6$ hydrocarbon radicals which may be substituted by cyano, hydroxy, halogen, $C_1$∿$_6$ alkoxy, $C_1$∿$_6$ alkoxy substituted by $C_1$∿$_6$ alkoxy, $C_2$∿$_6$ alkenyloxy, $C_2$∿$_6$ alkynyloxy, $C_3$∿$_8$ alkynyloxycarbonyloxy, $C_1$∿$_6$ alkylthio, $C_1$∿$_6$ alkylsulfinyl, $C_1$∿$_6$ alkylsulfonyl, amino substituted by $C_1$∿$_6$ alkyl, hydroxyimino, $C_1$∿$_6$ alkoxyimino or $C_2$∿$_8$ alkoxycarbonyl; and

-B- represents -O-, -S-, -SO-, -$SO_2$- or -N(R')- (R' is hydrogen or $C_1$∿$_6$ alkyl); and

Y' represents same or different substituent(s) selected from a group consisting of satulated or unsaturated $C_1$∿$_6$ hydrocarbon radicals which may be substituted by halogen, cyano, $C_3$∿$_6$ cycloalkyl, $C_2$∿$_8$ alkylcarbonyloxy, $C_2$∿$_8$ alkylcarbonyl, $C_2$∿$_8$ alkoxycarbonyl, hydroxy, $C_1$∿$_6$ alkoxy, $C_1$∿$_6$ alkylthio, ureido or heterocyclic radical containing oxygen; and

a part of ⟵(B-Y)n represents bi-substitutive modified radical ⟵(B-Y⟶ selected from a group consisting of $C_1$∿$_3$ alkylenedioxy which may be substituted by $C_1$∿$_6$ alkoxy, $-O-(CH_2)_\ell-O(CH_2)_{\ell'}-$ and $-O-(CH_2)_\ell-O-CO-$(each of ℓ and ℓ' is a integer from 1 to 3); and

Y" represents same or different substituent(s) selected from a group consisting of satulated or unsaturated $C_1\sim_6$ hydrocarbon radicals which may be substituted by halogen, cyano, $C_3\sim_6$ cycloalkyl, $C_2\sim_8$ alkylcarbonyloxy, $C_2\sim_8$ alkylcarbonyl, $C_2\sim_8$ alkoxycarbonyl, hydroxy, $C_1\sim_6$ alkoxy, $C_1\sim_6$ alkylthio, ureido or heterocyclic radical containing oxygen; and

m' represents an integer from 0 to 4;

n" represents an integer from 1 to 5;

n' represents an integer from 0 to 4, with the proviso that $1\leq(m'+n'+n")\leq 5$; and

R represents a substituent selected from a group consisting of satulated or unsatulated $C_1\sim_{18}$ hydrocarbon radicals which may be substituted by halogen, cyano, $C_1\sim_6$ alkoxy, $C_1\sim_6$ alkylthio, $C_1\sim_6$ alkoxycarbonyl;

Hal is halogen; and

r' is $C_1\sim_4$ alkyl or phenyl which may be substituted by methyl; and

with the proviso that "C number ∿ number" represents the range of total carbon number of the substituent or radical directly following thereto.

European Patent Office

# EUROPEAN SEARCH REPORT

0132881
Application number
EP 84201035.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US - A - 4 237 168 (WALTER REIF-SCHNEIDER)<br>* Claims; column 1 *<br>-- | 1,24-26 | C 07 C 131/00<br>C 07 C 149/14<br>C 07 C 149/42<br>C 07 C 147/14<br>C 07 D 303/22<br>C 07 D 317/28<br>A 01 N 37/52 |
| A | DE - A1 - 2 717 437 (HOECHST AG)<br>* Claims *<br>-- | 1-3, 24-26 | |
| A | DE - A1 - 2 911 865 (CHEVRON RESEARCH CO.)<br>* Claim 1 *<br>-- | 1 | |
| A | EP - A1 - 0 024 747 (BAYER AG)<br>* Claims *<br>---- | 1,26 | |
| | | | TECHNICAL FIELDS SEARCHED (Int. Cl.4) |
| | | | C 07 C 131/00<br>C 07 C 123/00 |

The present search report has been drawn up for all claims

| Place of search | Date of completion of the search | Examiner |
|---|---|---|
| VIENNA | 18-09-1984 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document